# EUROPEAN PATENT SPECIFICATION

(11) **EP 3 144 031 B1**
(45) Date of publication and mention of the grant of the patent: **18.07.2018**
(21) Application number: 15793376.3
(22) Date of filing: 02.04.2015
(51) Int. Cl.: A61M 37/00, A61K 38/38

(54) **METHOD OF MANUFACTURING TRANSDERMAL ABSORPTION SHEET**
VERFAHREN ZUR HERSTELLUNG EINER TRANSDERMALEN ABSORPTIONSSCHICHT
PROCÉDÉ DE FABRICATION DE FEUILLE D'ABSORPTION TRANSDERMIQUE

(30) Priority: 15.05.2014 JP 2014101751
(43) Date of publication of application: 22.03.2017
(73) Proprietor: Fujifilm Corporation, Minato-ku Tokyo 106-8620 (JP)
(72) Inventor: WAKAMATSU, Satoshi, Ashigarakami-gun Kanagawa 258-8538 (JP); KABATA, Koki, Ashigarakami-gun Kanagawa 258-8538 (JP)
(74) Representative: Klunker IP Patentanwälte PartG mbB
(86) International application number: PCT/JP2015/060453
(87) International publication number: WO 2015/174160

(56) References cited:
- EP-A1- 2 283 809
- JP-A- 2011 012 050
- JP-A- 2011 245 055
- US-A1- 2008 269 685

## Description

### {Technical Field}

The present invention relates to a method of manufacturing a transdermal absorption sheet according to the preamble of claim 1 and a transdermal absorption sheet.

### {Background Art}

In recent years, transdermal absorption sheets in which needle-shaped projections (also referred to as fine needles or microneedles) containing a drug are formed have been used in order to deliver the drug into the skin. In general, the transdermal absorption sheet is pressed to the skin to insert the needle-shaped projections into the skin, thereby delivering the drug in the needle-shaped projections into the skin.

As a method of manufacturing the transdermal absorption sheet, PTL 1 discloses a method using a mold having a through hole formed in the bottom of a needle-shaped recess, in which a base solution that is a polymer solution containing no drug is filled after a drug solution that is a polymer solution containing a drug is filled to the needle-shaped recess of the mold and the drug solution in the needle-shaped recess is dried.

A method of manufacturing a transdermal absorption sheet according to the preamble of claim 1 is known from EP 2 283 809 A1.

JP 2011 245055 A discloses a transdermal absorption sheet in which a solution obtained by dissolving a polymer into a solvant is put into a mold having needle-shaped recesses. After drying and shrinking, there is obtained a scaled-down shape of the needle-shaped recesses.

US 2008/269685 A1 discloses a transdermal absorption sheet comprising plural layers arranged parallel to a base, wherein at least two of the plural layers comprise different polymers.

### {Citation List}

### {Patent Literature}

{PTL 1} Japanese Patent Application Laid-Open No. 2011-078617

### {Summary of Invention}

### {Technical Problem}

When filling the needle-shaped recess with the drug and the base solution, in some cases air bubbles occurring in a solution sending system such as a liquid feed tank, pipes, a pipe connection portion and nozzles or gases in the needle-shaped recess before the filling, are sucked in the drug solution and the base solution. In a case of manufacturing the transdermal absorption sheet in this state, the air bubble possibly remains in the needle-shaped projections in the transdermal absorption sheet to generate air bubble defects. When the air bubble defect occurs, a variation occurs in the drug content in the needle-shaped projections depending upon a size of the air bubble, possibly bringing in a variation in dosage amount due to the air bubble or instability of the drug effects. In addition, the needle-shaped projection is impaired in a peeling-off step, and the needle-shaped projection may thus not bear a load during puncture and be broken, thereby resulting in a puncture error. It is difficult to distinguish between the air bubble defects and foreign objects such as insoluble particles, which leads to a problem in quality. If the air bubble is determined as the foreign object, the yield ratio is decreased.

In PTL 1, the needle-shape recess is filled with the drug solution by removing air from a through hole provided in a tip end of a mold. However, even if the through hole is formed in the tip end of the needle-shaped recess, since the through hole is sealed off by the drug solution after being filled with the drug solution, the air cannot be sufficiently removed from the needle-shaped recess through the through hole. Thus, there is a possibility that the air bubble is taken into the needle-shaped recess.

For complete removal of the air bubbles, there are conceived some methods such as a method in which the solution is coated in a reduced-pressure environment, a method in which pressurization is performed for defoaming before the drug solution is dried, and the like. The reduced-pressure filling can suppress the mixing of the air bubble in an environment in a high degree of vacuum, but there are some cases where density and viscosity of the drug solution increase due to evaporation of water components so as to impair uniformity in a filling amount in a plurality of needle-shaped recesses. In a case of the drug solution filling in an environment in a low degree of vacuum in a range less subjected to the influence of the evaporation of water components, the suppression of the air bubble generation becomes insufficient. The increased-pressure defoaming requires a pressurized vessel and makes the process complicated, leading to a reduction in productivity.

The present invention is made in view of the circumstances, and an object of the present invention is to provide a method of manufacturing a transdermal absorption sheet and a transdermal absorption sheet that can suppress generation of air bubbles.

### {Solution to Problem}

According to the present embodiment, a method of manufacturing a transdermal absorption sheet includes, in this order: comprising, in this order: a drug solution filling step of filling needle-shaped recesses of a mold, the needle-shaped recesses being arranged two-dimensionally, with a drug solution that is a polymer solution containing a predetermined amount of a drug; a drug solution drying step of drying the drug solution filled in the needle-shaped recesses to form a first layer containing a predetermined amount of a drug; a base solution filling step of filling the needle-shaped recesses on the first layer with a base solution that is a polymer solution not containing a predetermined amount of a drug; a base solution drying step of drying the base solution to form a second layer not containing a predetermined amount of a drug on the first layer; and a peeling-off step of peeling off the first layer and the second layer from the mold, wherein each step of at least from the drug solution filling step to the base solution drying step is performed in an environment with a temperature of 1 °C to 10 °C.

At least one step of the drug solution filling step and the base solution drying step is preferably performed in an environment with a relative humidity of 1%RH to 59%RH.

The method of manufacturing a transdermal absorption sheet preferably includes at least one of a step of deaerating the drug solution before the drug solution filling step and a step of deaerating the base solution before the base solution filling step.

The needle-shaped recess of the mold preferably has a through hole at a tip end thereof.

In the drug solution drying step, the drying is preferably performed in a calm state.

In the drug solution filling step, a nozzle that discharges the drug solution is scanned on the mold in a state where the nozzle is pressed to the mold, and the needle-shaped recesses are filled with the drug solution from the nozzle while a pressing force of the nozzle to the mold is controlled.

In the drug solution filling step, a nozzle that discharges the drug solution is scanned on the mold in a state where the nozzle is pressed to the mold, and the needle-shaped recesses are filled with the drug solution from the nozzle while a push-in distance (pressing distance) of the nozzle to the mold is controlled.

In the drug solution filling step, the amount of the drug solution to be filled is preferably equal to a total volume of the needle-shaped recesses.

The drug is preferably a peptide, a protein, a nucleic acid, a polysaccharide, a vaccine, a medical compound belonging to a water-soluble low-molecular compound, or a cosmetic component.

### {Advantageous Effects of Invention}

According to the method of manufacturing the transdermal absorption sheet it is possible to suppress the generation of air bubbles.

### {Brief Description of Drawings}

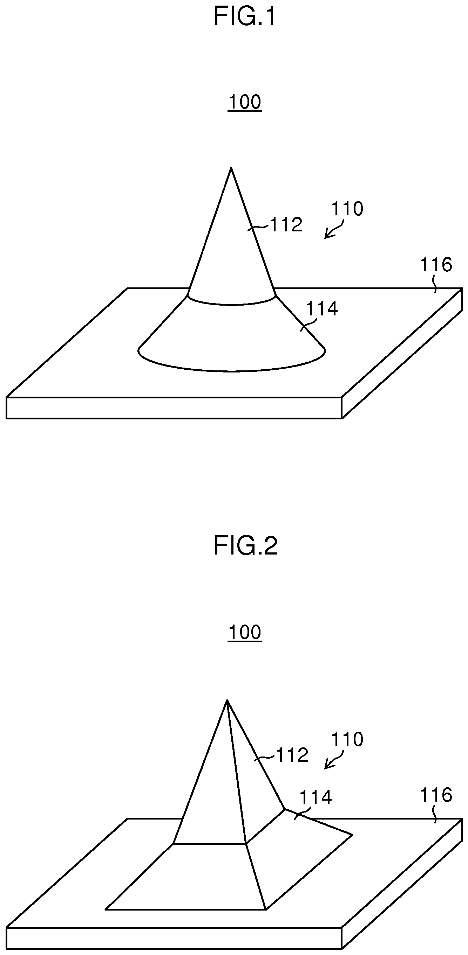

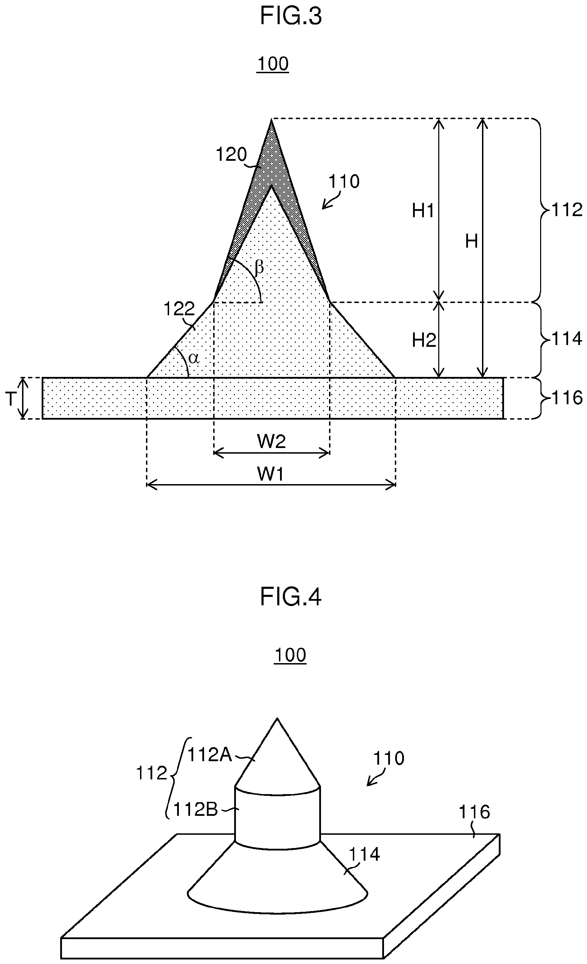

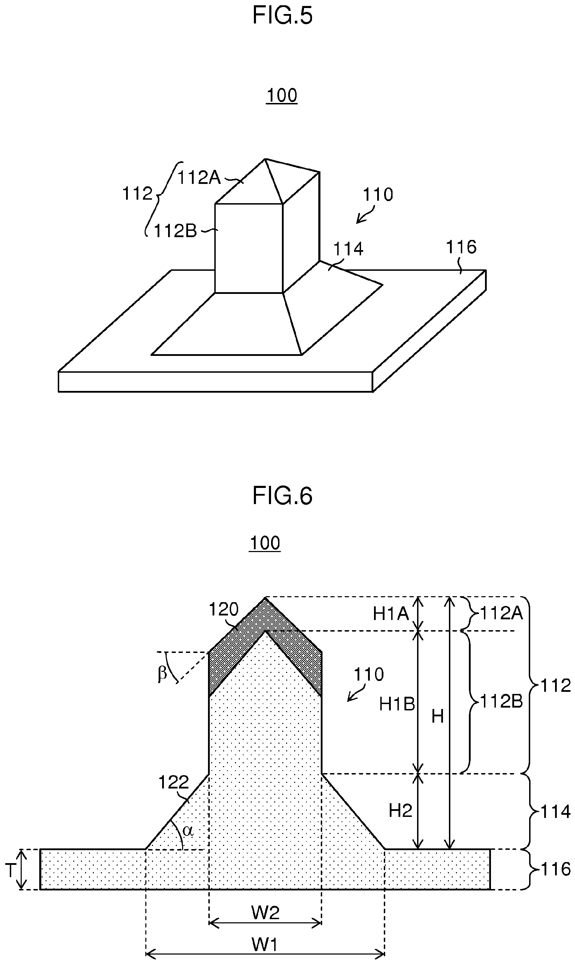

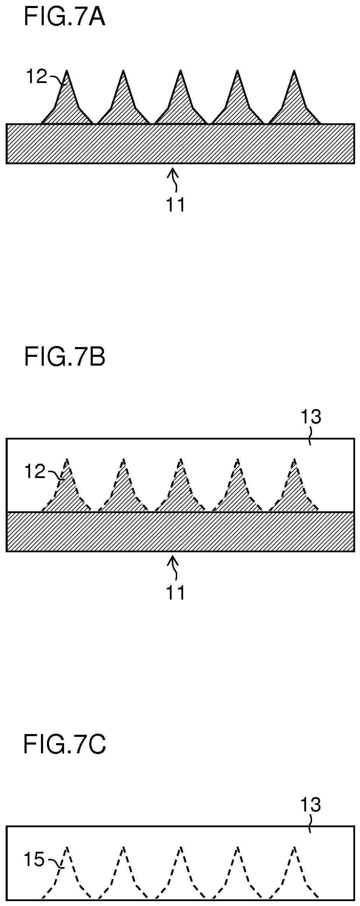

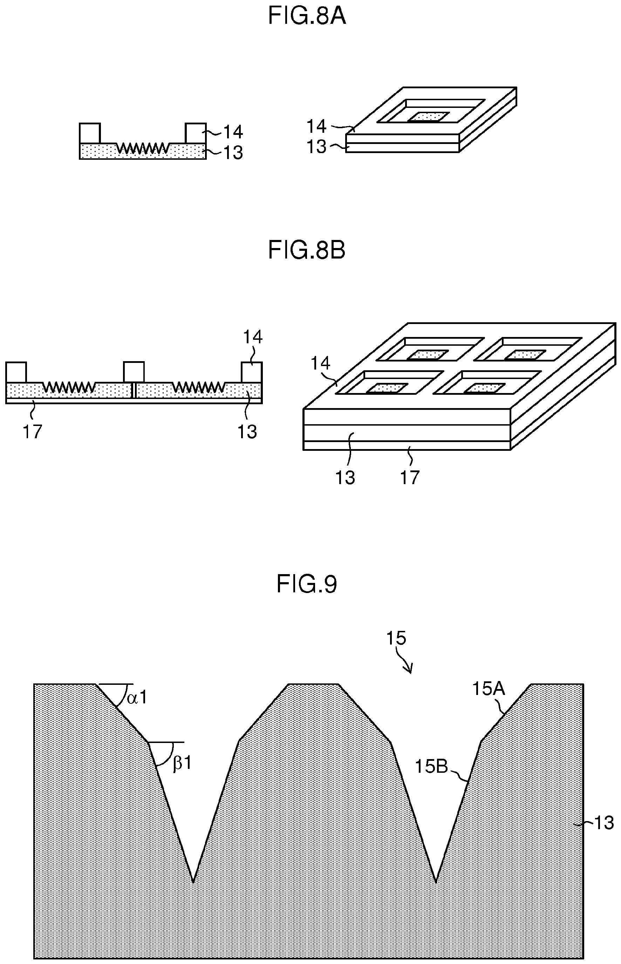

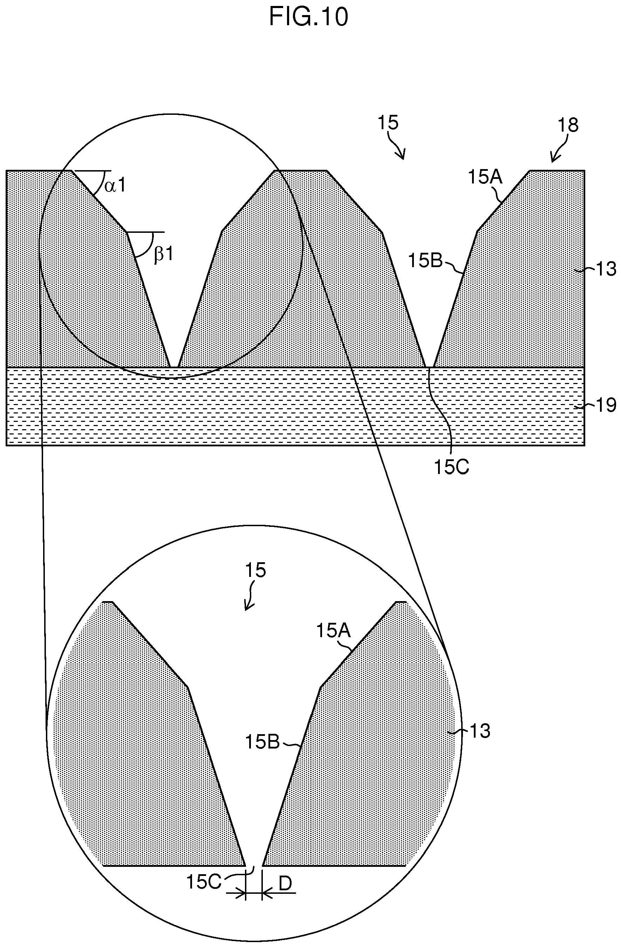

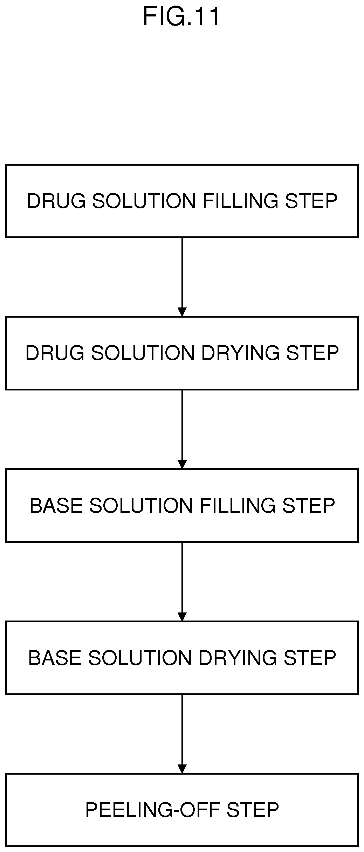

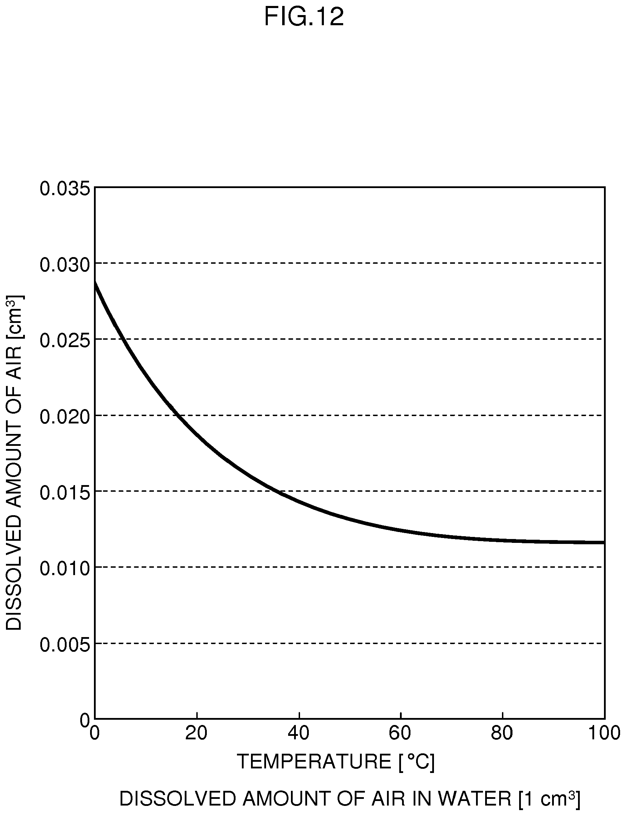

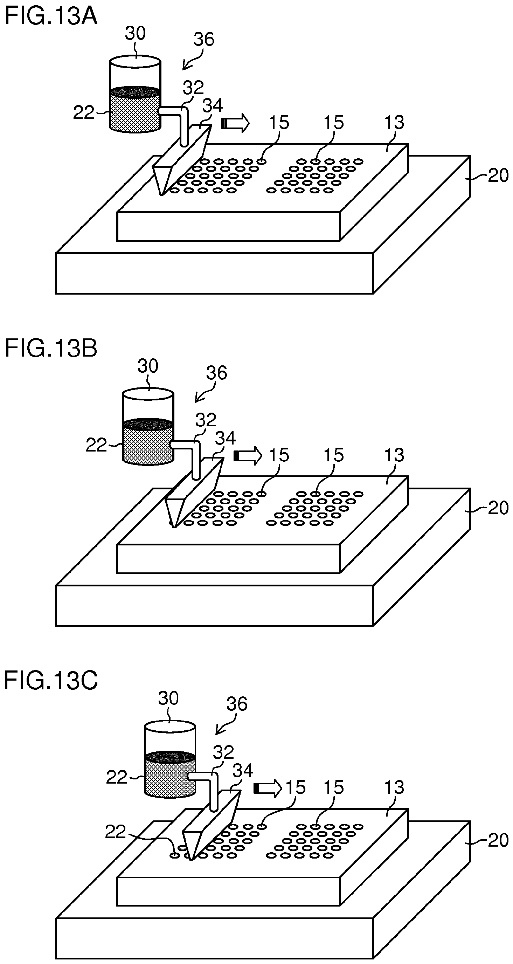

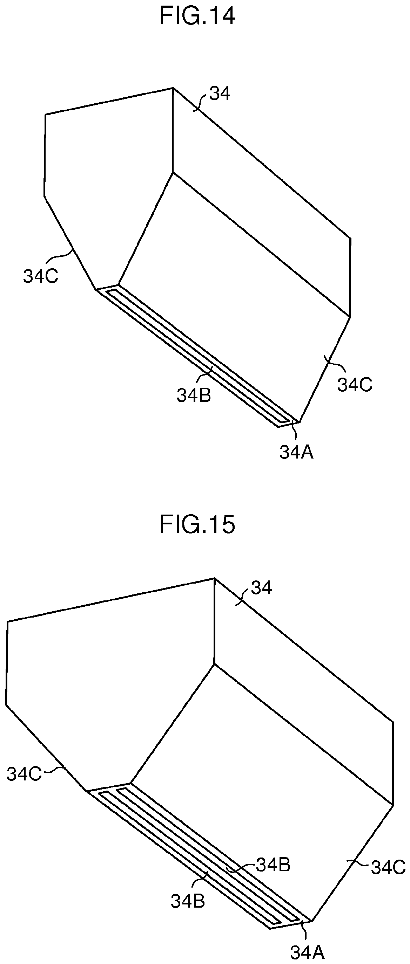

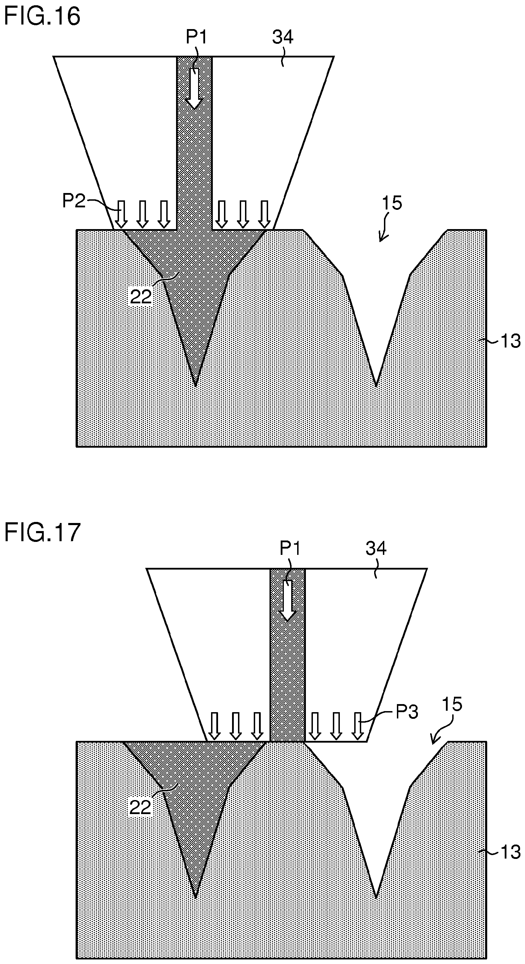

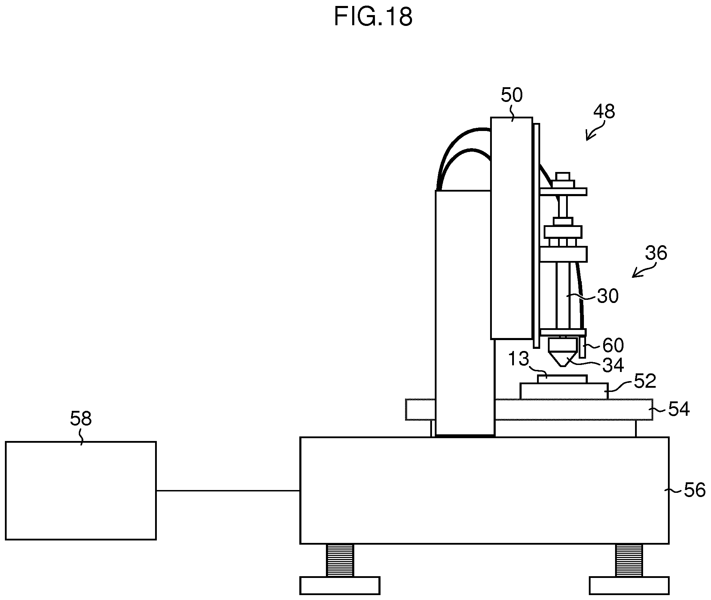

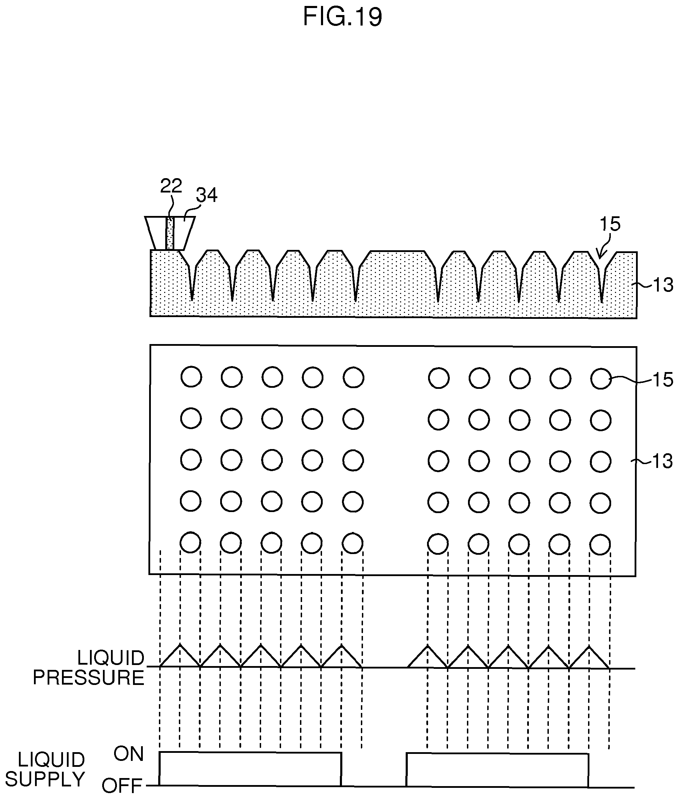

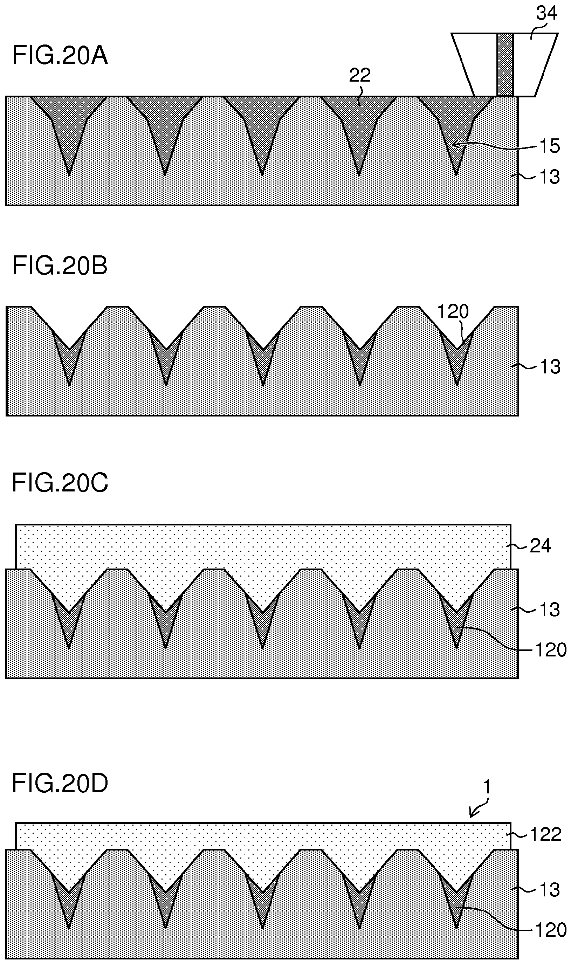

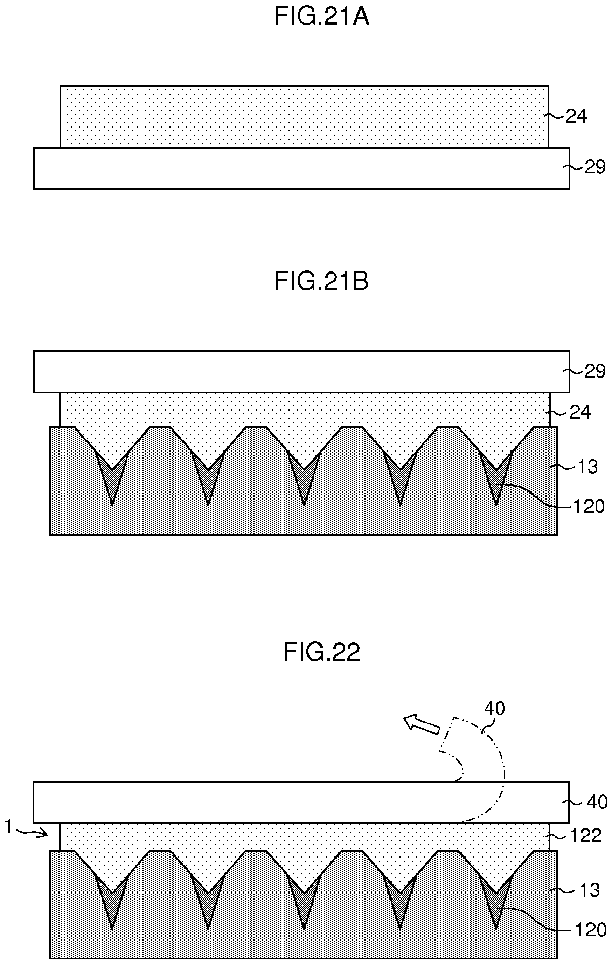

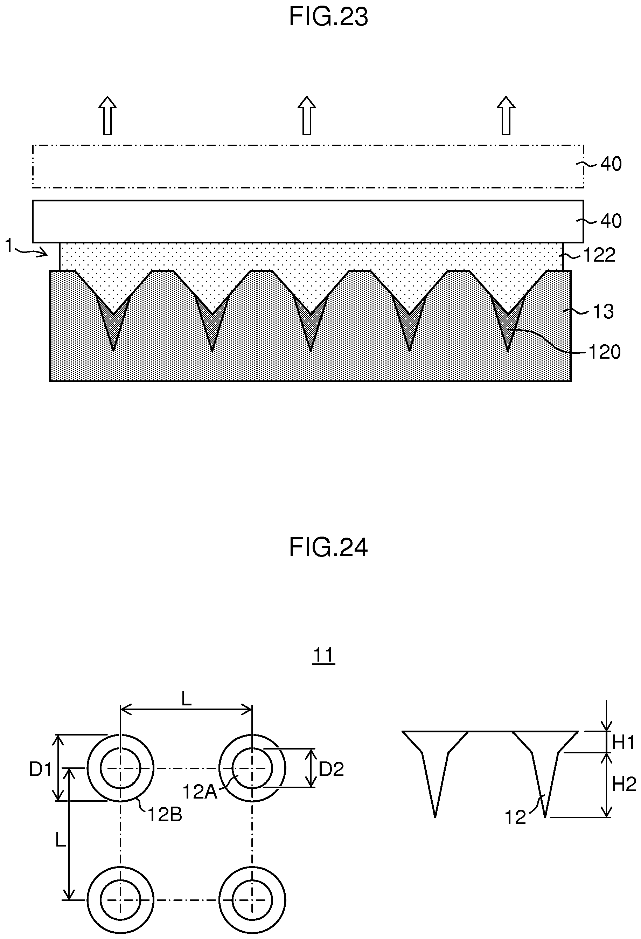

{Figure 1} Figure 1 is a perspective view of a transdermal absorption sheet having a needle-shaped projection.
{Figure 2} Figure 2 is a perspective view of a transdermal absorption sheet having a needle-shaped projection having a different shape.
{Figure 3} Figure 3 is a cross-sectional view of the needle-shaped projection of the transdermal absorption sheet illustrated in Figures 1 and 2.
{Figure 4} Figure 4 is a perspective view of a transdermal absorption sheet having a needle-shaped projection having a different shape.
{Figure 5} Figure 5 is a perspective view of a transdermal absorption sheet having a needle-shaped projection having a different shape.
{Figure 6} Figure 6 is a cross-sectional view of the needle-shaped projection of the transdermal absorption sheet illustrated in Figures 4 and 5.
{Figure 7A} Figure 7A is a drawing illustrating a step in a method of manufacturing a mold.
{Figure 7B} Figure 7B is a drawing illustrating a step in the method of manufacturing a mold.
{Figure 7C} Figure 7C is a drawing illustrating a step in the method of manufacturing a mold.
{Figure 8A} Figure 8A shows a cross-sectional view and a perspective view of a mold on which a frame is provided.
{Figure 8B} Figure 8B shows a cross-sectional view and a perspective view of molds on which a frame is provided.
{Figure 9} Figure 9 is a partially enlarged view of the mold manufactured in Figures 7A to 7C.
{Figure 10} Figure 10 is a partially enlarged view of the mold manufactured in Figures 7A to 7C.
{Figure 11} Figure 11 is a flow diagram of a method of manufacturing a transdermal absorption sheet.
{Figure 12} Figure 12 is a schematic configuration diagram of a liquid supplying device.
{Figure 13A} Figure 13A is a schematic diagram illustrating a step of filling the needle-shaped recesses of the mold with a drug solution.
{Figure 13B} Figure 13B is a schematic diagram illustrating the step of filling the needle-shaped recesses of the mold with a drug solution.
{Figure 13C} Figure 13C is a schematic diagram illustrating the step of filling the needle-shaped recesses of the mold with a drug solution.
{Figure 14} Figure 14 is a perspective view illustrating a tip end of a nozzle.
{Figure 15} Figure 15 is a perspective view illustrating a tip end of a different nozzle.
{Figure 16} Figure 16 is a partially enlarged view of a tip end of the nozzle and the mold during filling.
{Figure 17} Figure 17 is a partially enlarged view of the tip end of the nozzle and the mold during scanning.
{Figure 18} Figure 18 is a schematic configuration diagram of a drug solution filling apparatus.
{Figure 19} Figure 19 is an explanation drawing illustrating a relationship between the liquid pressure in the nozzle and the supply of the solution containing a drug.
{Figure 20A} Figure 20A is a schematic diagram illustrating a part of another manufacturing process of a transdermal absorption sheet.
{Figure 20B} Figure 20B is a schematic diagram illustrating a part of the other manufacturing process step of manufacturing a transdermal absorption sheet.
{Figure 20C} Figure 20C is a schematic diagram illustrating a part of the other manufacturing process step of manufacturing a transdermal absorption sheet.
{Figure 20D} Figure 20D is a schematic diagram illustrating a part of the other manufacturing process step of manufacturing a transdermal absorption sheet.
{Figure 21A} Figure 21A is a schematic diagram illustrating a part of the other manufacturing process step of manufacturing a transdermal absorption sheet.
{Figure 21B} Figure 21B is a schematic diagram illustrating a part of the other manufacturing process step of manufacturing a transdermal absorption sheet.
{Figure 22} Figure 22 is an explanation drawing illustrating a peeling-off step.
{Figure 23} Figure 23 is an explanation drawing illustrating another peeling-off step.
{Figure 24} Figure 24 shows a plan view and a side view of an original plate.

### {Description of Embodiments}

Hereinafter, preferred embodiments of the invention will be described with reference to the accompanying drawings. The invention will be described with the following preferred embodiments. Modifications can be made by many methods without departing from the scope of the invention, and embodiments other than the embodiments can be used. Accordingly, all of the modifications within the scope of the invention are included in the claims.

Here, in the drawings, the parts represented by the same references are the same elements having the same functions. In this description, in a case where a numerical value range is expressed using the form of "... to ...", the numerical values of the upper limit and the lower limit shown in the form of "... to ..." are also included in the numerical value range.

A transdermal absorption sheet manufactured in the present embodiment will be described. Figures 1 and 2 each illustrates a needle-shaped projection 110 (also referred to as a fine needle or a microneedle) that is a partially enlarged view of a transdermal absorption sheet 100.

The transdermal absorption sheet 100 delivers a drug into the skin by being attached to the skin. As illustrated in Figure 1, the transdermal absorption sheet 100 has a tapered-shaped needle portion 112, a frustum portion 114 connected to the needle portion 112, and a flat plate-shaped sheet portion 116 connected to the frustum portion 114. The tapered-shaped needle portion 112 and the frustum portion 114 configure the needle-shaped projection 110.

A plurality of frustum portions 114 is formed on a front surface of the sheet portion 116 (only one frustum portion 114 is shown in Figure 1). Among two end surfaces of the frustum portion 114, an end surface (lower base) having a larger area is connected to the sheet portion 116. Among the two end surfaces of the frustum portion 114, an end surface (upper base) having a smaller area is connected to the needle portion 112. That is, among the two end surfaces of the frustum portion 114, an end surface in a direction in which it is separated from the sheet portion 116 has a smaller area. Since a surface having a larger area in the needle portion 112 is connected to the end surface having a smaller area in the frustum portion 114, the needle portion 112 has a gradually tapered shape in a direction in which it is separated from the frustum portion 114.

In Figure 1, the frustum portion 114 has a truncated circular cone shape, and the needle portion 112 has a cone shape. The shape of a tip end of the needle portion 112 can be appropriately changed to a curved surface having a radium of curvature of 0.01 µm to 50 µm, a flat surface, or the like in accordance with the degree of insertion of the needle portion 112 into the skin.

Figure 2 illustrates a needle-shaped projection 110 having a different shape. In Figure 2, the frustum portion 114 has a truncated quadrangular pyramid shape, and the needle portion 112 has a quadrangular pyramid shape.

Figure 3 is a cross-sectional view of the transdermal absorption sheet 100 illustrated in Figures 1 and 2. As illustrated in Figure 3, a first layer 120 containing a predetermined amount of a drug and a second layer 122 not containing a predetermined amount of a drug configure the transdermal absorption sheet 100. Here, the expression "containing a predetermined amount of a drug" means containing the drug in an amount such that medicinal effects are exhibited when puncture is performed on a body surface. The expression "not containing a predetermined amount of a drug" means that the drug is not contained in an amount such that medicinal effects are exhibited, and the range of the amount of the drug includes a range from 0, indicating that the drug is not contained at all, to an amount in which medicinal effects are not exhibited. The first layer 120 containing a drug is formed at a tip end (the tip end of the needle portion 112) of the needle-shaped projection 110. The drug can be efficiently delivered into the skin by forming the first layer 120 at the tip end of the needle-shaped projection 110. Hereinafter, the expression "containing a predetermined amount of a drug" will be referred to as "containing a drug", and the expression "not containing a predetermined amount of a drug" will be referred to as "not containing a drug", if necessary.

The second layer 122 is formed in a portion excluding the first layer 120 in the needle portion 112. The second layer 122 configures the frustum portion 114. The second layer 122 configures the sheet portion 116. Distribution of the first layer 120 and the second layer 122 which constitute the needle portion 112, the frustum portion 114, and the sheet portion 116 can be optionally set.

A thickness T of the sheet portion 116 is in a range of 10 µm to 2,000 µm, and preferably in a range of 10 µm to 1,000 µm. A width W1 of the portion (lower base) in which the frustum portion 114 and the sheet portion 116 are in contact with each other is in a range of 100 µm to 1,500 µm, and preferably in a range of 100 µm to 1,000 µm. A width W2 of the portion (upper base) in which the frustum portion 114 and the needle portion 112 are in contact with each other is in a range of 100 µm to 1,500 µm, and preferably in a range of 100 µm to 1,000 µm. The width W1 and the width W2 satisfy the relationship of W1> W2 within the above numerical value range.

A height H of the needle-shaped projection 110 is in a range of 100 µm to 2,000 µm, and preferably in a range of 200 µm to 1,500 µm. In addition, H1/H2 that is a ratio of a height H1 of the needle portion 112 to a height H2 of the frustum portion 114 is in a range of 1 to 10, and preferably in a range of 1.5 to 8. The height H2 of the frustum portion 114 is preferably in a range of 10 µm to 1,000 µm.

An angle α formed between a side surface of the frustum portion 114 and a surface parallel to the front surface of the sheet portion 116 is in a range of 10° to 60°, and preferably in a range of 20° to 50°. An angle β formed between a side surface of the needle portion 112 and a surface parallel to the upper base of the frustum portion 114 is in a range of 45° to 85°, and preferably in a range of 60° to 80°.

The angle β is preferably equal to or larger than the angle α. This is because the needle-shaped projection 110 is easily inserted into the skin.

Figures 4 and 5 each illustrates a needle-shaped projection 110 having a different shape. In the transdermal absorption sheet 100 illustrated in Figures 1 and 4 and the transdermal absorption sheet 100 illustrated in Figures 2 and 5, the frustum portions 114 have the same shape, and the needle portions 112 have different shapes. Each of the needle portions 112 illustrated in Figures 4 and 5 has a tapered-shaped needle-shaped portion 112A and a tubular body portion 112B. A bottom surface of the needle-shaped portion 112A and an end surface of the body portion 112B are connected to each other. Among end surfaces of the body portion 112B, an end surface that is not connected to the needle-shaped portion 112A is connected to the upper base of the frustum portion 114.

The needle-shaped portion 112A and the body portion 112B illustrated in Figure 4 have a cone shape and a columnar shape, respectively. The needle-shaped portion 112A and the body portion 112B illustrated in Figure 5 have a quadrangular pyramid shape and a quadrangular prism shape, respectively.

Since the needle portion 112 has the body portion 112B, the needle portion 112 has a shape having a constant width in a direction away from the frustum portion 114. The needle-shaped portion 112A of the needle portion 112 is gradually tapered in a direction away from the body portion 112B. In the tubular body portion 112B, two end surfaces thereof opposed to each other have almost the same area. The needle portion 112 has a tapered shape as a whole. The shape of a tip end of the needle portion 112 can be appropriately changed to have a curved surface with a radium of curvature of 0.01 µm to 50 µm, a flat surface, or the like in accordance with the degree of insertion of the needle portion 112 into the skin.

Figure 6 is a cross-sectional view of the transdermal absorption sheet 100 illustrated in Figures 4 and 5. As illustrated in Figure 6, a first layer 120 containing a drug and a second layer 122 not containing a drug configure the transdermal absorption sheet 100. The first layer 120 containing a drug is formed at a tip end (the tip end of the needle portion 112) of the needle-shaped projection 110. The drug can be efficiently delivered into the skin by forming the first layer 120 at the tip end of the needle-shaped projection 110.

The second layer 122 is formed in a portion excluding the first layer 120 in the needle portion 112. The second layer 122 configures the frustum portion 114. The second layer 122 configures the sheet portion 116. Distribution of the first layer 120 and the second layer 122 configuring the needle portion 112, the frustum portion 114, and the sheet portion 116 can be optionally set.

Each of a thickness T of the sheet portion 116, a width W1 of the lower base of the frustum portion 114, a width W2 of the upper base of the frustum portion 114, a height H of the needle-shaped projection 110, and a height H2 of the frustum portion 114 can be adjusted to the same length as each of the corresponding portions of the transdermal absorption sheet 100 illustrated in Fig. 3. H1/H2 that is a ratio of a height H1 of the needle portion 112, that is, H1A+H1B, to the height H2 of the frustum portion 114 can be adjusted to the same ratio as that of the transdermal absorption sheet 100 illustrated in Fig. 3.

H1B/H1A that is a ratio of a height H1B of the body portion 112B to a height H1A of the needle-shaped portion 112A is in a range of 0.1 to 4, and preferably in a range of 0.3 to 2.

An angle α formed between a side surface of the frustum portion 114 and a surface parallel to the front surface of the sheet portion 116 is in a range of 10° to 60°, and preferably in a range of 20° to 50°. An angle β formed between a side surface of the needle-shaped portion 112A and a surface parallel to the end surface of the body portion 112B is in a range of 45° to 85°, and preferably in a range of 60° to 80°.

The angle β is preferably equal to or larger than the angle α. This is because the needle-shaped projection 110 is easily inserted into the skin.

In the present embodiment, the transdermal absorption sheets 100 having the needle portions 112 illustrated in Figures 1, 2, 4, and 5 are shown, but the transdermal absorption sheet 100 is not limited to these shapes.

### (Mold)

Figures 7A to 7C are process drawings of the production of a mold (form).

As illustrated in Figure 7A, first, an original plate for producing a mold for manufacturing a transdermal absorption sheet is produced.

There are two methods for producing the original plate 11. A first method includes applying a photoresist to a Si substrate, exposing it to exposure light, and developing it. In addition, etching by reactive ion etching (RIE) or the like is performed to produce a plurality of projections 12, each having the same shape as the needle-shaped projection of the transdermal absorption sheet, in arrays on a front surface of the original plate 11. Here when etching such as RIE is performed in order to form the projections 12 on the front surface of the original plate 11, the projections 12 can be formed by performing etching from an oblique direction while rotating the Si substrate.

As a second method, there is a method including processing a metal substrate such as Ni using a cutting tool such as a diamond bit to produce a plurality of projections 12 in arrays on the front surface of the original plate 11.

Next, as illustrated in Figure 7B, a mold 13 is produced using the original plate 11. In order to produce a normal mold 13, a method using Ni electroforming is used. Since the original plate 11 has the projections 12 having a conical shape or pyramid shape (for example, quadrangular pyramid) with a sharp tip end, the shape is accurately transferred to the mold 13, and the mold 13 can be peeled off from the original plate 11. Four methods are considered for manufacturing at a low cost.

A first method is a method in which a silicone resin obtained by adding a curing agent to polydimethylsiloxane (PDMS, for example, SYLGARD 184 manufactured by Dow Corning Corporation) is poured into the original plate 11 and cured by being heat-treated at 100°C, and then the mold 13 is peeled off from the original plate 11. A second method is a method in which a UV-curable resin that is curable by ultraviolet irradiation is poured into the original plate 11 and irradiated with ultraviolet rays in a nitrogen atmosphere, and then the mold 13 is peeled off from the original plate 11. A third method is a method in which a material obtained by dissolving a plastic resin such as polystyrene or polymethylmethacrylate (PMMA) in an organic solvent is poured into the original plate 11 which has been coated with a peeling agent, and is dried to volatilize the organic solvent for curing, and then the mold 13 is peeled off from the original plate 11. A fourth method is a method in which a reversed product is made by Ni electroforming.

Accordingly, a mold 13 is produced in which needle-shaped recesses 15 having a reversal shape of the projections 12 of the original plate 11 are arranged two-dimensionally. The mold 13 produced in this manner is illustrated in Figure 7C. In any of the three methods, the mold 13 can be easily produced any number of times.

Figures 8A and 8B are drawings in which a frame 14 is installed on the mold 13 manufactured in Figure 7C. Figure 8A is a drawing in which the frame is installed on the periphery of the front surface of one mold 13. Figure 8B is a drawing in which a frame 14 is provided on the periphery and on the inside of a plurality of molds 13 which have been joined together. By providing the frame 14, it is possible to prevent a solution in which a polymer resin is dissolved (hereinafter, also referred to as "polymer solution") from flowing to the outside of the mold 13 when forming a transdermal absorption sheet with a desired thickness.

Here, a height difference between the mold 13 and the frame 14 is preferably 50 µm to 10 mm. The forms of Figures 8A and 8B have a configuration in which the mold 13 and the frame 14 can be separated from each other. However, it may have a configuration in which the mold and the frame are formed integrally with each other. In a case of a separation-type configuration, the frame 14 can be detached in a drying step or a peeling-off step after a filling step.

As illustrated in Figure 8B, the plurality of molds 13 are joined together on a substrate 17 using an adhesive. The frame 14 is installed on the periphery and on the inside of the molds 13 which have been joined together.

Figure 9 is a partially enlarged view of the mold 13. The needle-shaped recess 15 is provided with a tapered inlet portion 15A that is narrowed in a depth direction from the front surface of the mold 13 and a tip end recess 15B that is tapered in the depth direction. An angle α1 of the taper of the inlet portion 15A basically corresponds to the angle α formed between the side surface of the frustum portion and the sheet portion in the transdermal absorption sheet. The angle β1 of the taper of the tip end recess 15B basically corresponds to the angle β formed between the side surface of the needle portion and the upper base of the frustum portion.

Figure 10 illustrates a more preferred embodiment of a mold composite 18 in performing a method of manufacturing a transdermal absorption sheet. As illustrated in Figure 10, the mold composite 18 is formed of a mold 13 in which a through hole 15C is formed at a tip end of a needle-shaped recess 15 and a gas permeable sheet 19 that is bonded to the side of the through hole 15C of the mold 13 and is made of a material that is gas permeable, but is not liquid permeable. Through the through hole 15C, the tip end of the needle-shaped recess 15 communicates with the atmosphere via the gas permeable sheet 19. The expression "tip end of the needle-shaped recess 15" means a side that is tapered in a depth direction of the mold 13 and is opposite to a side from which a drug solution or a base solution is filled.

Using such a mold composite 18, only the air present in the needle-shaped recess 15 can be removed from the needle-shaped recess 15 via the through hole 15C without permeation of a transdermal absorption material solution filled in the needle-shaped recess 15. Transferability in a case where the shape of the needle-shaped recess 15 is transferred to the transdermal absorption material is improved, and thus it is possible to form a sharper needle-shaped projection.

A diameter D of the through hole 15C is preferably in a range of 1 to 50 µm. By adjusting the diameter within this range, air bleeding is easily performed, and the tip end portion of the needle-shaped projection of the transdermal absorption sheet is allowed to have a sharp shape. As the gas permeable sheet 19 made of a material that is gas permeable, but is not liquid permeable, for example, POREFLON (trademark, Sumitomo Electric Industries, Ltd.) can be preferably used.

As the material for use in the mold 13, an elastic material or a metallic material can be used. Among these, an elastic material is preferred, and a material having high gas permeability is more preferred. The oxygen permeability representative for the gas permeability is preferably greater than 1 × 10⁻¹² (mL/s·m·Pa), and more preferably greater than 1 × 10⁻¹⁰ (mL/s·m·Pa). By adjusting the gas permeability within the above range, the air present in the needle-shaped recess 15 of the mold 13 can be removed from the mold 13. It is possible to manufacture a transdermal absorption sheet with few defects. Specific examples of such a material include materials obtained by melting a silicone resin (for example, SYLGARD 184 or 1310ST), a UV-curable resin, or a plastic resin (for example, polystyrene or polymethylmethacrylate (PMMA)) and materials obtained by dissolving any of the above resins in a solvent. Among these, silicone rubber-based materials can be preferably used since these are durable in transfer by repeated pressurization and have good peelability from the material. Examples of the metallic material include Ni, Cu, Cr, Mo, W, Ir, Tr, Fe, Co, MgO, Ti, Zr, Hf, V, Nb, Ta, α-aluminum oxide, zirconium oxide, stainless steel (STAVAX), and alloys thereof. As the material of the frame 14, the same material as that of the mold 13 can be used.

### (Polymer Solution)

A polymer solution that is used in the present embodiment and is a solution in which a polymer resin is dissolved will be described.

In the present embodiment, the polymer solution containing a predetermined amount of a drug is referred to as a polymer solution containing a drug or a solution containing a drug, and the polymer solution not containing a predetermined amount of a drug is referred to as a polymer solution not containing a drug or a solution not containing a drug, if necessary. In addition, the polymer solution containing a predetermined amount of drug is referred to as a drug solution, and the polymer solution not containing a predetermined amount of a drug is referred to as a base solution. Whether a predetermined amount of a drug is contained is determined based on whether medicinal effects are exhibited when the solution is punctured on a body surface. Accordingly, the expression "containing a predetermined amount of a drug" means that the drug is contained in an amount such that medicinal effects are exhibited when punctured on a body surface. In addition, the expression "not containing a predetermined amount of a drug" means that the drug is not contained in an amount such that medicinal effects are exhibited, and the range of the amount of the drug includes a range from 0, indicating that the drug is not contained at all, to an amount in which medicinal effects are not exhibited.

As the material of the resin polymer used in the polymer solution, a biocompatible resin is preferably used. As such a resin, saccharides such as glucose, maltose, pullulan, chondroitin sulfate, sodium hyaluronate, and hydroxyethyl starch, proteins such as gelatin, polylactates, and biodegradable polymers such as a lactic acid-glycollic acid copolymer are preferably used. Among these, gelatin-based materials have adhesiveness with many base materials and have a high gel strength as a material to be gelated. Thus, the gelatin-based materials can be preferably used during a peeling-off step to be described below since these can be closely attached to the base material to allow the polymer sheet to be peeled off from the mold using the base material. The concentration of the resin is preferably adjusted such that 10% to 50% by mass of the resin polymer is contained in the polymer solution not containing a drug, though the concentration depends on the material. In addition, a solvent used for dissolution may be other than hot water as long as it has volatility, and methyl ethyl ketone (MEK), alcohol, or the like can be used. In the solution in which the polymer resin is dissolved, a drug to be supplied to the inside of a body can concurrently be dissolved in accordance with the application. The polymer concentration of the polymer solution containing a drug (in a case where the drug itself is a polymer, the concentration of the polymer excluding the drug) is preferably 0% to 30% by mass.

As a method of preparing the polymer solution, in a case where a water-soluble polymer (gelatin or the like) is used, a water-soluble powder may be dissolved in water and a drug may be added thereto. Otherwise, a water-soluble polymer powder may be added and dissolved in a liquid in which a drug is dissolved. In a case where a material that is difficult to dissolve in water is used, it may be dissolved by heating. The temperature can be appropriately selected depending on the type of the polymer material, but the material is preferably heated at a temperature of approximately 60°C or lower. The viscosity of the solution in which the polymer resin is dissolved is preferably 100 Pa·s or less, and more preferably 10 Pa·s or less in the solution containing a drug. The viscosity is preferably 2,000 Pa·s or less, and more preferably 1,000 Pa·s or less in the solution not containing a drug. The solution is easily injected into the needle-shaped recess of the mold by appropriately adjusting the viscosity of the solution in which the polymer resin is dissolved. For example, the viscosity of the solution in which the polymer resin is dissolved can be measured using a tube-type viscometer, a drop-type viscometer, a rotational viscometer, or a vibration-type viscometer.

### (Drug)

The drug that is contained in the polymer solution is not limited as long as it functions as a drug. Particularly, the drug is preferably selected from a peptide, a protein, a nucleic acid, a polysaccharide, a vaccine, a medical compound belonging to a water-soluble low-molecular compound, or a cosmetic component.

### (Method of Manufacturing Transdermal Absorption Sheet)

As illustrated in Figure 11, the method of manufacturing a transdermal absorption sheet according to the present embodiment includes at least five steps of a drug solution filling step, a drug solution drying step, a base solution filling step, a base solution drying step, and a peeling-off step in this order. Each step of the drug solution filling step, the drug solution drying step, the base solution filling step, and the base solution drying step is performed in an environment with a temperature of 1°C to 10°C.

The inventors have earnestly investigated suppression of generation of air bubbles in the drug solution and the base solution. As a result, in an environment with a temperature of 1°C to 10°C, the air bubble taken into the drug solution and/or the base solution can be dissolved in the polymer solution constituting the drug solution and/or the base solution so as to reduce the air bubble defect.

A size of the air bubble depends on a shape of the needle-shaped recess 15, but the air bubbles with a diameter of several 10 µm to 100 µm are primarily generated. The size of the air bubble corresponds to 0.001 to 2.0% of the volume of the needle-shaped recess 15. The inventors have found that in an environment with a temperature of 1°C to 10°C, the air bubble in this level of a size can be dissolved in the polymer solution. Thus, the present invention has been achieved.

By performing the steps where the air bubble is soluble in the polymer solution, that is, each step of the drug solution filling step, the drug solution drying step, the base solution filling step, and the base solution drying step in an environment with a temperature of 1°C to 10°C, the generation of the air bubble is suppressed. Since the air bubble is dissolved in the polymer solution, it is possible to maintain uniformity of the filling amount. In addition, since the air bubble is suppressed by forming an environment with a temperature of 1°C to 10°C, the steps are simple. Thus, it possible to improve the yield ratio and productivity of the transdermal absorption sheet.

For performing each step in an environment with a temperature of 1°C to 10°C, for example, all of the five steps may be set in a thermostatic chamber or in a thermostatic tank or only a part of the steps may be installed in a thermostatic chamber or in a thermostatic tank. A temperature range thereof is preferably 1°C to 10°C, more preferably 2°C to 8°C.

Moreover, by performing each step of the drug solution filling step, the drug solution drying step, the base solution filling step, and the base solution drying step in an environment with a temperature of 1°C to 10°C, modification of the drug can be suppressed to improve quality of the transdermal absorption sheet.

In each step, only a region where the drug solution and/or the base solution remains in a liquid state may be set in an environment with a temperature of 1°C to 10°C. It is possible to locally cool only a region where the drug solution and/or the base solution remains in a liquid state, using a chiller or a peltier device, for example.

Figure 12 is a graph illustrating a relationship between the temperature and the solubility of the gas in water of 1 cm³. The vertical axis represents a volume of the gas dissolved in water under 1 atm (1 atm is equivalent to 1013 hPa), and the horizontal axis represents the temperature. The air bubble defect in the transdermal absorption sheet is caused by air taken during manufacturing steps. From this graph, the solution of the air in water of 1 cm³ becomes greater as the temperature becomes lower. In a case where the temperature is 10°C, the dissolved amount of air in water of 1 cm³ is approximately 0.023 cm³ in a gas state under 1 atm. A general volume of the needle-shaped recess of the mold is approximately 0.03 mm³, and a volume of the air bubble with a diameter of 100 µm is approximately 0.00052 mm³. A volume of the air bubble to a volume of the needle-shaped recess is 0.017 times, and is smaller than 0.023. Therefore, the air bubble can be dissolved in the polymer solution to suppress the generation of the air bubble. It is possible to suppress the generation of the air bubble defect due to the air bubble.

A temperature between the respective steps is preferably set to an environment with a temperature of 1°C to 10°C. In a case where the temperature between the respective steps exceeds 10°C, in some cases gases dissolved in the polymer solution become air bubbles. Therefore, the temperature between the respective steps is preferably set to an environment with a temperature equal to or less than 15°C, further, equal to or less than 10°C preferably. In addition, it is preferable to make the time for passing between the respective steps as short as possible, and in a case where the temperature between the respective steps exceeds 10°C, particularly it is preferable to shorten the time.

### (Drug Solution Filling Step)

A method of manufacturing a transdermal absorption sheet using the mold 13 will be described. As illustrated in Fig. 13A, the mold 13 having the needle-shaped recesses 15 which are arranged two-dimensionally is disposed on a base 20. In the mold 13, two sets of the needle-shaped recesses 15 are formed, and in each of the sets, 5 × 5 needle-shaped recesses are arranged two-dimensionally. There is prepared a liquid supply device 36 which includes a liquid feed tank 30 storing a drug solution 22 that is a polymer solution containing a predetermined amount of a drug, a pipe 32 connected to the liquid feed tank 30, and a nozzle 34 connected to a tip end of the pipe 32. The drug solution 22 is discharged from a tip end of the nozzle 34.

Figure 14 shows a schematic perspective view of the tip end portion of the nozzle. As illustrated in Figure 14, the nozzle 34 is provided with a lip portion 34A having a flat surface on the tip end side thereof, a slit-shaped opening portion 34B, and two inclined surfaces 34C expanding in a direction away from the opening portion 34B along the lip portion 34A. Through the slit-shaped opening portion 34B, for example, it is possible to simultaneously fill a plurality of needle-shaped recesses 15 configuring one row with the drug solution 22. The size (length and width) of the opening portion 34B is appropriately selected in accordance with the number of needle-shaped recesses 15 to be filled at one time.

By increasing the length of the opening portion 34B, the number of needle-shaped recesses 15 to be filled with the drug solution 22 at one time can be increased. Accordingly, productivity can be improved.

Figure 15 shows a schematic perspective view of a tip end portion of another nozzle. As illustrated in Figure 15, a nozzle 34 is provided with a lip portion 34A having a flat surface on the tip end side thereof, two slit-shaped opening portions 34B, and two inclined surfaces 34C expanding in a direction away from the opening portions 34B along the lip portion 34A. Through the two opening portions 34B, for example, it is possible to simultaneously fill a plurality of needle-shaped recesses 15 configuring two rows with the drug solution 22 containing a drug.

As the material for use in the nozzle 34, an elastic material or a metallic material can be used. Examples thereof include TEFLON (registered trademark), stainless steel (SUS), and titanium.

The filling step will be described with reference to Figure 13A to Figure 13C. As illustrated in Figure 13A, the opening portion 34B of the nozzle 34 is positionally adjusted above the needle-shaped recesses 15. The nozzle 34 that discharges the drug solution 22 is pressed to the mold 13, and thus the lip portion 34A of the nozzle 34 and the front surface of the mold 13 are brought into contact with each other. As illustrated in Figure 13B, the drug solution 22 is supplied to the mold 13 from the liquid supply device 36, and thus the needle-shaped recesses 15 are filled with the drug solution 22 from the opening portion 34B of the nozzle 34. In the present embodiment, a plurality of needle-shaped recesses 15 configuring one row is filled with the drug solution 22 simultaneously. However, the invention is not limited thereto, and the needle-shaped recesses 15 can be filled one by one. In addition, using the nozzle 34 illustrated in Figure 15, the plurality of needle-shaped recesses 15 configuring the plurality of rows can be simultaneously filled with the drug solution 22 every plural rows.

In a case where the mold 13 is made of a gas permeable material, the drug solution 22 can be sucked by suction from a rear surface of the mold 13, and thus it is possible to promote the filling of the needle-shaped recesses 15 with the drug solution 22.

After the filling step illustrated in Figure 13B, as illustrated in Figure 13C, the liquid supply device 36 is relatively scanned in a direction perpendicular to a length direction of the opening portion 34B while the lip portion 34A of the nozzle 34 and the front surface of the mold 13 are brought into contact with each other. The nozzle 34 performs scanning on the mold 13, and the nozzle 34 is moved to a needle-shaped recess 15 that is not filled with the drug solution 22. The opening portion 34B of the nozzle 34 is positionally adjusted above the needle-shaped recess 15. In the present embodiment, the example in which the nozzle 34 is scanned has been described, but the mold 13 may be scanned.

Since the nozzle 34 is scanned on the mold 13 with the contact between the lip portion 34A of the nozzle 34 and the front surface of the mold 13, the nozzle 34 can scrape off the drug solution 22 remaining on the front surface other than the needle-shaped recesses 15 of the mold 13. It is possible to prevent the drug solution 22 containing a drug from remaining on regions other than the needle-shaped recesses 15 of the mold 13. In the present embodiment, the nozzle 34 is disposed such that the inclined surfaces 34C are positioned to be perpendicular to the scanning direction shown by the arrow. Accordingly, the nozzle 34 can perform smooth scanning on the mold 13.

In order to reduce damage on the mold 13 and suppress deformation by compression of the mold 13 as much as possible, it is preferable that the degree of pressing the nozzle 34 to the mold 13 during scanning is controlled. For example, it is preferable that a pressing force of the nozzle 34 to the mold 13 and a push-in distance of the nozzle 34 to the mold 13 are controlled. In addition, in order to prevent the drug solution 22 from remaining on regions other than the needle-shaped recesses 15 of the mold 13, it is desirable that the material of at least one of the mold 13 and the nozzle 34 is flexible and elastically deformed.

By repeating the filling step of Figure 13B and the moving step of Figure 13C, the needle-shaped recesses 15 which are two-dimensionally arranged in 5 × 5, are filled with the drug solution 22. When the needle-shaped recesses 15 which are two-dimensionally arranged in 5 × 5 has been filled with the drug solution 22, the liquid supply device 36 is moved to adjacent needle-shaped recesses 15 which are two-dimensionally arranged in 5 × 5, and the filling step of Figure 13B and the moving step of Figure 13C are repeated. The adjacent needle-shaped recesses 15 which are two-dimensionally arranged in 5 × 5 are also filled with the drug solution 22.

Regarding the above-described filling step and scanning step, an embodiment (1) in which the needle-shaped recesses 15 are filled with the drug solution 22 while the nozzle 34 is scanned, may be applied, or an embodiment (2) in which the nozzle 34 is temporarily stopped on the needle-shaped recesses 15 during the scanning of the nozzle 34 and filled with the drug solution 22, and after the filling, the nozzle 34 is scanned again, may be applied. The lip portion 34A of the nozzle 34 is pressed to the front surface of the mold 13 between the filling step and the scanning step. The amount of the drug solution 22 to be discharged from the liquid supply device 36 is preferably the same as the total volume of the plurality of needle-shaped recesses 15 of the mold 13. The drug solution 22 is prevented from remaining on the front surface other than the needle-shaped recesses 15 of the mold 13 to be filled, and the loss of the drug can be reduced.

Figure 16 is a partially enlarged view of the tip end of the nozzle 34 and the mold 13 while the needle-shaped recess 15 is filled with the drug solution 22. As illustrated in Figure 16, it is possible to promote the filling of the needle-shaped recesses 15 with the drug solution 22 by applying a pressure P1 into the nozzle 34. Furthermore, when the needle-shaped recesses 15 are filled with the drug solution 22, a pressing force P2 for bringing the nozzle 34 into contact with the front surface of the mold 13 is preferably equal to or greater than the pressure P1 in the nozzle 34. By satisfying the equation of "pressing force P2 ≥ pressure P1," it is possible to suppress leakage of the drug solution 22 from the needle-shaped recess 15 to the front surface of the mold 13.

Figure 17 is a partially enlarged view of the tip end of the nozzle 34 and the mold 13 during the movement of the nozzle 34. When the nozzle 34 is relatively scanned with respect to the mold 13, it is preferable that a pressing force P3 for bringing the nozzle 34 into contact with the front surface of the mold 13 is smaller than the pressing force P2 for bringing the nozzle 34 into contact with the front surface of the mold 13 during the filling. This is for reducing damage on the mold 13, and suppressing deformation of the mold 13 by compression.

The lip portion 34A of the nozzle 34 is preferably parallel to the front surface of the mold 13. The posture of the nozzle 34 may be controlled by providing a joint driving mechanism at a mounting portion of the nozzle 34.

The pressing force and/or the push-in distance of the nozzle 34 to the mold 13 is/are preferably controlled by driving the nozzle 34 in a Z-axis direction along the front surface shape of the mold 13. Fig. 18 is a schematic configuration diagram of a drug solution filling apparatus 48 capable of controlling the pressing force and/or the push-in distance. The drug solution filling apparatus 48 includes: a liquid supply device 36 that has a liquid feed tank 30 storing a drug solution and a nozzle 34 mounted on the liquid feed tank 30; a Z-axis driving unit 50 that drives the liquid feed tank 30 and the nozzle 34 in the Z-axis direction; a suction base 52 for placing the mold 13 thereon; a X-axis driving unit 54 that drives the suction base 52 in a X-axis direction; a stand 56 that supports the device; and a control system 58.

A case of controlling a pressing force to be constant will be described. The Z-axis driving unit 50 brings the nozzle 34 close to the mold 13 up to Z-coordinates in which a desired pressing force is obtained. While the nozzle 34 brought into contact with the mold 13 is scanned by the X-axis driving unit 54, the drug solution 22 is discharged while Z-axis coordinate control is performed such that the pressing force becomes constant. A method for measuring the contact pressure is not particularly limited, but for example, various load cells can be used, for example, under the suction base 52 or in place of the suction base 52. The load cell means a measuring instrument capable of measuring a force for compression in a thickness direction. The pressing force is an arbitrary pressure within a range of 1 to 1,000 kPa against the mold 13, and is preferably controlled to be constant.

A case of controlling a push-in distance to be constant will be described. Before contact with the nozzle 34, the front surface shape of the mold 13 is measured in advance. While the nozzle 34 brought into contact with the mold 13 is scanned by the X-axis driving unit 54, the drug solution 22 is discharged while feeding back a value obtained by offsetting the Z-axis coordinate to the Z-axis driving unit 50 such that a push-in distance to the front surface of the mold 13 becomes a desired push-in distance depending on the front surface shape of the mold.

The shape measuring method is not particularly limited. For example, an optical measuring instrument such as a non-contact-type laser displacement meter 60 or a contact-type (probe-type) step profiler can be used. Furthermore, the posture of the nozzle 34 in a slit's longitudinal direction may be controlled along the front surface shape of the mold 13. The push-in distance is preferably controlled within a range of 1% to 15% with respect to the thickness of the mold 13. Through the operation with the control of the distance between the nozzle 34 and the mold 13 along the shape of the mold 13 in the Z-axis direction by the Z-axis driving unit 50, the compression deformation rate is uniformized, and thus the accuracy of the filling amount can be improved.

Regarding the control of the pressing force and the push-in distance, the pressing force is preferably controlled in a case where the push-in distance is small, and the push-in distance is preferably directly controlled in a case where the push-in distance is large.

Figure 19 is an explanation drawing illustrating a relationship between the liquid pressure in the nozzle and the supply of the solution containing a drug. As illustrated in Figure 19, the supply of the drug solution 22 is started before the nozzle 34 is positioned above the needle-shaped recesses 15. The reason for this is to securely fill the needle-shaped recesses 15 with the drug solution 22. Until the filling of the plurality of needle-shaped recesses 15 composed of 5 × 5 is completed, the drug solution 22 is continuously supplied to the mold 13. The supply of the drug solution 22 to the mold 13 is stopped before the nozzle 34 is positioned above needle-shaped recesses 15 in the fifth row. It is possible to prevent the drug solution 22 from overflowing from the needle-shaped recesses 15. The liquid pressure in the nozzle 34 increases in a region where the nozzle 34 is not positioned above the needle-shaped recesses 15 when the supply of the drug solution 22 is started. On the other hand, when the nozzle 34 is positioned above the needle-shaped recesses 15, the needle-shaped recesses 15 are filled with the drug solution 22, and the liquid pressure in the nozzle 34 decreases. The liquid pressure repeatedly changes.

When the filling of the plurality of needle-shaped recesses 15 composed of 5 × 5 is completed, the nozzle 34 is moved to an adjacent needle-shaped recesses 15 composed of 5 × 5. Regarding the liquid supply, the supply of the drug solution 22 is preferably stopped when the nozzle is moved to the adjacent needle-shaped recesses 15 composed of 5 × 5. There is a distance between the needle-shaped recesses 15 in the fifth row and the needle-shaped recesses 15 in the next first row. In a case where the drug solution 22 is continuously supplied while the nozzle 34 is scanned between them, the liquid pressure in the nozzle 34 may excessively increase. As a result, the drug solution 22 may flow to regions other than the needle-shaped recesses 15 of the mold 13 from the nozzle 34. In order to suppress this problem, the supply of the drug solution 22 is preferably stopped.

When the filling of the drug solution 22 is performed, it is preferable that the tip end of the nozzle 34 is cleaned before being used. This is because the accuracy of the filling amount of the drug solution 22 is reduced in a case where a material adheres to the surface of the lip portion 34A of the nozzle 34 before filling. In general, wiping using non-woven cloth is performed for cleaning. During wiping, the cleaning can be effectively performed in a case where non-woven cloth is permeated with water, a solvent, or the like.

After the filling of the drug solution 22, the drug solution may remain on the front surface of the mold 13 when the nozzle 34 is separated from the mold 13. By performing suck back control for suction of the drug solution 22 from the opening portion 34B of the nozzle 34 after completion of the filling of the needle-shaped recesses 15, a surplus of the drug solution 22 can be sucked, and the liquid remaining on the front surface of the mold 13 can thus be reduced.

In the present embodiment, the drug solution filling step is performed in an environment with a temperature of 1°C to 10°C. Accordingly, an air bubble having a diameter of approximately several tens µm to 100 µm can be dissolved in the polymer solution constituting the drug solution 22 even in a case where an air bubble is taken into the drug solution 22 in the liquid feed tank 30, the pipe 32, the pipe connection portion, the nozzle 34, or the like, or a case where the air in the needle-shaped recess 15 is taken into the drug solution 22 when the needle-shaped recess 15 of the mold 13 is filled with the drug solution 22.

In the drug solution filling step, the drug solution can be sucked from the through hole 15C using the mold 13 illustrated in Figure 10 to fill the needle-shaped recess 15 with the drug solution 22.

When the filling of the needle-shaped recesses 15 with the drug solution 22 is completed, the process proceeds to the drug solution drying step, the base solution filling step, the base solution drying step, and the peeling-off step.

As illustrated in Figure 20A, the needle-shaped recesses 15 of the mold 13 are filled with the drug solution 22 from the nozzle 34 in the drug solution filling step. The drug solution filling step is performed using the above-described method.

### (Drug Solution Drying Step)

As illustrated in Figure 20B, in the drug solution drying step, the drug solution 22 is dried and solidified, and thus first layers 120 containing a drug are formed in the needle-shaped recesses 15.

The drug solution drying step is a step of drying the drug solution 22 filled in the needle-shaped recesses 15 of the mold 13 and localizing the drug solution at the tip ends of the needle-shaped recesses 15. In the present embodiment, the drug solution drying step is performed in an environment with a temperature of 1°C to 10°C. In a case where the drug solution drying step is performed in an environment with a temperature higher than 10°C, the gas (air) dissolved in the polymer solution of the drug solution 22 in the drug solution filling step may turn into an air bubble due to an increase in temperature. In a case where the amount of the gas dissolved is small, no air bubble is generated. However, the increase in temperature may cause an unexpected air bubble defect. Accordingly, the generation of the air bubble defect can be reduced by performing the drug solution drying step in an environment with a temperature of 1°C to 10°C.

In addition, by optimizing the drying rate with the control of the temperature and humidity conditions of the drug solution drying step, it is possible to reduce the adhesion of the drug solution 22 to a wall surface of the mold 13 of the needle-shaped recesses 15, and the drying proceeds while the drug solution 22 is collected at the tip ends of the needle-shaped recesses 15 by drying. For example, in an environment of 23°C/40 to 60%RH, the drying rate (drying speed) is high, and thus the drug solution 22 may adhere to a wall surface of the mold 13 of the needle-shaped recesses 15 and it is difficult to localize the drug solution 22 at the tip ends of the needle-shaped recesses 15.

In the present embodiment, the drying rate of the drug solution 22 is low since the drug solution drying step is performed in an environment with a temperature of 1°C to 10 °C. Accordingly, the drug solution 22 can be localized at the tip ends of the needle-shaped recesses 15 without adhesion of the drug solution 22 to a wall surface of the mold 13. In the drug solution drying step in an environment with a temperature of 1°C to 10°C, in a case where the humidity is high, the drying rate of the drug solution 22 is reduced, and thus it leads to a reduction in productivity. Accordingly, in a case where the drug solution drying step is performed in an environment with a temperature of 1 °C to 10 °C, an environment with a relative humidity of 1% to 59% is preferably provided, and an environment with a relative humidity of 21% to 39% is more preferably provided. In an environment with a relative humidity range of 1% to 59% at a temperature of 1°C to 10°C, it is possible to achieve high productivity and the localization of the drug solution 22 at the tip ends of the needle-shaped recesses 15 at the same time.

In order to provide an environment with a relative humidity of 1% to 59%, for example, the drug solution drying step is preferably performed in a thermostatic chamber or a thermostatic tank having a humidity adjustment function.

The drug solution 22 is preferably dried in a calm state in the drug solution drying step. Uneven drying occurs in a case where the drug solution 22 is directly exposed to nonuniform wind. This is because, in a portion exposed to strong wind, the drying rate may be increased, the drug solution 22 may adhere to a wall surface of the mold 13, and thus the localization of the drug solution 22 at the tip ends of the needle-shaped recesses 15 may be disturbed.

In order to realize the drying in a calm state, for example, a windshield is preferably installed. The windshield is installed so as not to directly expose the mold 13 to wind. As the windshield, a physical obstacle such as a lid, a hood, a screen, a fence, or the like is preferably installed since this is a simple method. In addition, when the windshield is installed, a vent hole or the like is preferably secured such that the installation space for the mold 13 is not in an enclosed state. In a case where the installation space is in an enclosed state, water vapor in the enclosed space may be saturated, and the drying of the drug solution 22 may not proceed. The vent hole is preferably formed such that the passage of vapor is possible, and is more preferably covered with a water vapor permeable film or the like to stabilize the air flow in the windshield. The drying time is appropriately adjusted in consideration of the shape of the needle-shaped recess 15, the arrangement and the number of the needle-shaped recesses 15, the type of the drug, the filling amount and the concentration of the drug solution 22, and the like.

The calm state refers to a case where the wind speed is 0.5 m/s or less, including a state in which there is no wind at all. The reason why the wind speed is within this range is that uneven drying rarely occurs.

In the drug solution drying step, the drug solution 22 is solidified by being dried, and is reduced in size compared with that when the filling with the drug solution 22 is performed. Accordingly, in the peeling-off step, the first layer 120 can be easily peeled off from the needle-shaped recess 15 of the mold 13.

### (Base Solution Filling Step)

Next, as illustrated in Figure 20C, a base solution 24 that is a polymer solution not containing a predetermined amount of a drug is applied on the first layer 120 containing a predetermined amount of a drug using a dispenser, and the needle-shaped recesses 15 are filled with the base solution 24. The base solution 24 in an amount larger than the spaces of the needle-shaped recesses 15 is filled in the needle-shaped recesses. Bar coating, spin coating, coating using a spray, or the like may be applied in place of coating using the dispenser. Since the first layer 120 containing the drug has been solidified by drying, it is possible to suppress the drug contained in the first layer 120 from being diffused to the base solution 24.

In the present embodiment, the base solution filling step is performed in an environment with a temperature of 1°C to 10°C. Accordingly, even if the air bubble is taken in the base solution 24 in the liquid feed tank, the pipe, the pipe connection portion, the dispenser, or the like, or the air in the needle-shaped recess 15 is taken in the base solution 24 when the needle-shaped recess 15 of the mold 13 is filled with the base solution 24, it is possible to dissolve the air bubble in a size with a diameter of approximately several 10 to 100 µm in the polymer solution constituting the base solution 24.

Next, another embodiment of the base solution filling step will be described. As illustrated in Figure 21A, the base solution 24 that is a polymer solution not containing a drug is applied on a separate support 29. The support 29 is not limited, and for example, polyethylene, polyethylene terephthalate, polycarbonate, polypropylene, acrylic resin, triacetylcellulose, or the like can be used. Next, as illustrated in Fig. 21B, the base solution 24 formed on the support 29 is stacked (overlaid) on the mold 13 in which the first layer 120 has been formed in the needle-shaped recess 15. Therefore, the needle-shaped recess 15 is filled with the base solution 24.

In the present embodiment, the base solution filling step includes two steps, that is, applying the base solution 24 on the support 29 and stacking the base solution 24 on the support 29, on the mold 13. Any of the steps is performed in an environment with a temperature of 1°C to 10°C. It is possible to dissolve the air bubble in a size with a diameter of approximately several 10 to 100 µm in the polymer solution constituting the base solution 24.

In the present embodiment, the base solution is filled on the first layer in the needle-shaped recess 15 after forming the first layer 120. Since the first layer 120 has been solidified by drying, the air bubble contained in the base solution 24 can be removed through the surface of the needle-shaped recess 15 in the mold 13 via a clearance between the first layer 120 and the surface of the mold 13, and a pin hole of the first layer 120. When the mold used is the mold 13 having a through hole, the air bubble can be removed via the through hole.

On the other hand, a case where the base solution is filled on the drug solution without drying the drug solution 22 will be described. Since the drug solution 22 is in a liquid state, the passage of the gas such as a clearance or a pin hole is not present. Thus, there is no escape way of the air bubble. The route the gas in the base solution 24 has to pass is as follows: the gas in the base solution 24 is dissolved in the drug solution 22, and the dissolved gas diffuses, for example, from the through hole. This makes the time for air bubble removal longer. As a result, in a case when the drug solution 22 is not dried, since the passage of the gas is limited, in some cases, the gas cannot be completely dissolved and air bubble may remain even on a low temperature condition.

That is, removal of the air bubble from the base solution 24 becomes easier by forming the first layer 120 by drying the drug solution 22.

### (Base Solution Drying Step)

Next, as illustrated in Figure 20D, a second layer 122 not containing a predetermined amount of a drug is formed on the first layer 120 by drying and solidifying the base solution 24. A polymer sheet 1 having the first layer 120 and the second layer 122 is manufactured.

In the present embodiment, the base solution drying step is performed in an environment with a temperature of 1°C to 10°C. In a case where the base solution drying step is performed in an environment with a temperature higher than 10°C, the gas (air) dissolved in the polymer solution of the base solution 24 in the base solution filling step may turn into an air bubble due to an increase in temperature. In a case where the amount of the gas dissolved is small, no air bubble is generated. However, the increase in temperature may cause an unexpected air bubble defect. Accordingly, the generation of the air bubble defect can be reduced by performing the base solution drying step in an environment with a temperature of 1°C to 10°C.

In the base solution drying step in an environment with a temperature of 1°C to 10°C, in a case where the humidity is high, the drying rate (drying speed) of the base solution 24 is reduced, and thus it leads to a reduction in productivity. Accordingly, in a case where the base solution drying step is performed in an environment with a temperature of 1°C to 10°C, an environment with a relative humidity of 1% to 59% is preferably provided, and an environment with a relative humidity of 21% to 39% is more preferably provided. In an environment with a relative humidity range of 1% to 59% at a temperature of 1°C to 10°C, it is possible to improve productivity.

In order to provide an environment with a relative humidity of 1% to 59%, for example, the drug solution drying step is preferably performed in a thermostatic chamber or a thermostatic tank having a humidity adjustment function.

In order to promote the drying of the base solution 24, wind having a speed of 0.1 to 10 m/s is preferably blown. The base solution 24 does not contain a predetermined amount of a drug. Accordingly, even when uneven drying occurs, the influence thereof is small.

In the base solution drying step, the volume of the base solution 24 is reduced by drying. In a case where the base solution 24 is closely attached to the mold 13 during the drying, the reduction in volume occurs in the thickness direction of the sheet, and thus the thickness is reduced.

In a case where the base solution 24 is separated from the mold 13 during the drying, the polymer sheet 1 shrinks also in a plane direction. Accordingly, the polymer sheet 1 may be distorted or may curl. In a case where the polymer sheet 1 is separated from the mold 13 in a state in which the base solution 24 in the needle-shaped recesses 15 is not sufficiently dried, defects such as breaking or bending of the shape of the needle-shaped projections of the polymer sheet 1 may be easily generated. Accordingly, the polymer sheet 1 is preferably not separated from the mold 13 during the drying. In addition, in order to suppress curling, a layer that shrinks to the same extent as the front surface having the needle-shaped projections may be formed on the rear surface (the surface opposite to the surface having the needle-shaped projections formed therein) of the polymer sheet 1. For example, the same polymer solution as that on the front surface side is applied on the rear surface side to form a layer having a thickness whose curling suppression effect has been confirmed in advance.

### (Peeling-Off Step)

The method of peeling off the polymer sheet 1 from the mold 13 is not limited. It is desirable that the needle-shaped projections do not bend or are not broken during peeling-off. Specifically, as illustrated in Figure 22, a sheet-shaped base material 40 on which an adhesive layer having adhesive properties is formed is adhered to the polymer sheet 1, and then the base material 40 can be peeled off to be turned over from an end portion. However, in this method, the needle-shaped projections may bend. Therefore, it is possible to apply a method in which a sucker (not shown) is placed on the rear surface of the polymer sheet 1, and then vertically lift the polymer sheet while sucking it by air, as illustrated in Figure 23. A transdermal absorption sheet 100 is manufactured by peeling off the polymer sheet 1 from the mold 13.

Usually, in a case where a structure including a needle-shaped projection having a high aspect ratio is peeled off from the mold 13 as in the present embodiment, a strong stress is applied due to a large contact area between the projection and the mold. The fine needle that is the needle-shaped projection is broken and remains in the needle-shaped recess 15 without being peeled off from the mold 13. As a result, a transdermal absorption sheet to be produced has defects. In the present embodiment, the mold 13 is preferably made of a material that is very easily peelable. In addition, the mold 13 is made of a soft material having high elasticity, and thus the stress that is applied to the needle-shaped projection during peeling-off can be lessened.

### (Deaeration Step)

The drug solution 22 and/or the base solution 24 is/are preferably subjected to deaeration before the drug solution filling step and/or before the base solution filling step. Through deaeration, the air bubbles contained in the drug solution 22 and the base solution 24 can be removed before the filling of the needle-shaped recesses 15 of the mold 13. For example, in the deaeration step, air bubbles having a diameter of 100 µm to several millimeters are removed. By subjecting at least one of the drug solution 22 and the base solution 24 to deaeration, dissolution of the air bubble in the polymer solution can be promoted.

Examples of the deaeration method include (1) a method of exposing the drug solution 22 under a reduced pressure environment for 1 to 15 minutes, (2) a method of subjecting a container storing the drug solution 22 to ultrasonic vibration for 5 to 10 minutes, (3) a method of applying ultrasonic waves while exposing the drug solution 22 under a reduced pressure environment, and (4) a method of substituting the dissolved gas with helium by sending a helium gas into the drug solution 22. Any of the deaeration methods (1) to (4) can be applied to the base solution 24 also.

### {Examples}

Hereinafter, the invention will be described in more detail using examples of the invention. The materials, amounts, ratios, treatment contents, treatment procedures, and the like shown in the following examples can be appropriately changed without departing from the gist of the invention. The following specific examples are therefore to be considered in all respects as illustrative and not restrictive.

### <Example 1>

### (Production of Mold)

Projections 12 each having a needle-shaped structure in which a cone 12A having a diameter D2 of 300 µm and a height H2 of 500 µm was formed on a truncated circular cone 12B having a diameter D1 of 500 µm in a bottom surface and a height H1 of 150 µm as illustrated in Figure 24 were formed by grinding on a surface of a smooth Ni plate having a side of 40 mm so that the projections were arranged in two-dimensional arrays composed of 10 rows × 10 columns with a pitch L of 1,000 µm, and thus an original plate 11 was produced. On this original plate 11, a silicone rubber (SILASTIC MDX4-4210 manufactured by Dow Corning Corporation) film with a thickness of 0.6 mm was formed. The film was thermally cured in a state in which the tip end portions of the cones of the original plate 11 were projected by 50 µm from the film surface, and then the cured film was peeled off. Accordingly, a reversed silicone rubber product with through holes having a diameter of approximately 30 µm was produced. A flat portion except for a portion which had a side of 30 mm and in which needle-shaped recesses two-dimensionally arranged in 10 rows × 10 columns were formed in its center portion, was cut off from the reversed silicone rubber product, and the obtained portion was used as a mold. A surface in which the needle-shaped recesses had wide opening portions served as a front surface of the mold, and a surface having through holes (air bleed holes) having a diameter of 30 µm served as a rear surface of the mold.

### (Preparation of Polymer Solution Containing Drug)

Hydroxyethyl starch (manufactured by Fresenius Kabi) was dissolved in water to prepare an aqueous solution of 8%. To this aqueous solution, 2% by weight of human serum albumin (manufactured by Wako Pure Chemical Industries, Ltd.) as a drug was added to prepare a drug solution containing a drug. After the preparation, the drug solution was exposed in a reduced-pressure environment of 3 kPa for four minutes for deaeration.

### (Preparation of Polymer Solution Not containing Drug)

Chondroitin sulfate (manufactured by Maruha Nichiro Corporation) was dissolved in water to prepare an aqueous solution of 40% as a polymer solution not containing a drug, that is, a base solution. After the preparation, the base solution was exposed in a reduced-pressure environment of 3 kPa for four minutes for deaeration.

Hereinafter, the drug solution filling step to the base solution drying step were performed in an environment with a temperature of 5°C and with a relative humidity of 35%RH.

### (Drug Solution Filling Step and Drug Solution Drying Step)

A drug solution filling apparatus includes a driving unit that has a X-axis driving unit and Z-axis driving unit which control relative position coordinates of the mold and the nozzle, a liquid supply device (super small amount fixed-quantity dispenser SMP-III manufactured by Musashi Engineering, Inc.) on which the nozzle can be mounted, a suction base to which the mold is fixed, a laser displacement gauge (HL-C201A manufactured by Panasonic Corporation) that measures a front surface shape of the mold, a load cell (LCX-A-500N manufactured by Kyowa Electronic Instruments Co., Ltd.) that measures a nozzle pressing pressure, and a control system that controls the Z axis based on data of measurement values of the front surface shape and the pressing pressure.

A gas permeable film (POREFLON FP-010 manufactured by Sumitomo Electric Industries, Ltd.) having a side of 15 mm was placed on the flat suction base, and the mold was installed on the film such that the front surface of the mold was directed upward. The gas permeable film and the mold were fixed to the suction base by pressure reduction from a rear surface direction of the mold with a suction pressure of 90 kPa gauge pressure.

A SUS (stainless steel) nozzle having the shape illustrated in Figure 14 was prepared, and a slit-shaped opening portion having a length of 12 mm and a width of 0.2 mm was formed at the center of a lip portion having a length of 20 mm and a width of 2 mm. This nozzle was connected to the drug solution tank. The drug solution tank and the nozzle were filled with 3 mL of a solution containing a drug. The nozzle was adjusted such that the opening portion was parallel to a first row of a plurality of needle-shaped recesses formed in the front surface of the mold. The nozzle was pressed to the mold at a pressure (pressing force) of 0.14 kgf/cm² (1.4 N/cm²) at a position spaced apart from the first row with an interval of 2 mm therebetween in a direction opposite to a second row. With the nozzle being pressed, the nozzle was moved at 1 mm/sec in a direction perpendicular to a length direction of the opening portion while the Z axis was controlled such that the change in pressing force fell within ± 0.05 kgf/cm² (0.49 N/cm²). Simultaneously, by the liquid supply device, the solution containing a drug was discharged from the opening portion for 10 seconds at 0.31 µL/sec. The movement of the nozzle was stopped at a position spaced apart from a tenth row of the plurality of needle-shaped recesses arranged two-dimensionally with an interval of 2 mm from the tenth row in a direction opposite to a ninth row, and the nozzle was separated from the mold.

The mold filled with the drug solution was put and dried in a windshield (25 cm³) with an opening portion having a diameter of 5 mm. The windshield mentioned herein had a gas permeable film (POREFLON FP-010 manufactured by Sumitomo Electric Industries, Ltd.) mounted on the opening portion and was structured so as not to be directly exposed to wind to provide a calm state.

### (Base Solution Filling Step and Base Solution Drying Step)

A polymer solution (base solution) not containing a drug was applied to have a thickness of 75 µm on a polyethylene terephthalate (PET) which had been subjected to a hydrophilic treatment. The mold which had been filled with the drug solution was fixed to the suction base by suction. The front surface of the PET on which the base solution had been applied was disposed to face the front surface of the mold. Further, the pressure in a gap between the PET and the mold, and the pressure in a space on the side opposite to the mold of the PET were reduced for 2 minutes. After the pressure reduction, the PET and the mold were attached to each other by releasing only the space on the side opposite to the mold of the PET to atmospheric pressure. After the contact state was maintained for 10 minutes, a material obtained by attaching the PET and the mold to each other in an integrated manner was dried.

### (Peeling-Off Step)

The mold was peeled off from the polymer layer on the PET so as to be turned over from an end portion. On the PET, a transdermal absorption sheet with a three-dimensional arrangement structure that included a first layer containing a drug in which the human serum albumin was locally distributed at a tip end and a second layer not containing a drug was formed.

### (Evaluation)

The amount of air bubbles generated in the needle-shaped projection in the produced transdermal absorption sheet was evaluated. As an evaluation method, the transdermal absorption sheet was observed using a microscope (VHX-600 manufactured by Keyence Corporation). The presence or absence of air bubble generation was evaluated in a plurality of transdermal absorption sheets produced, and there was no transdermal absorption sheets in which air bubble was present in the needle-shaped projections of the transdermal absorption sheets.

### <Example 2>

### (Production of Mold)

A mold was produced in the same way with Example 1.

### (Preparation of Solution containing Drug)

A solution was prepared in the same way with Example 1.

### (Preparation of Solution not containing Drug)

A solution was prepared in the same way with Example 1.

Hereinafter, a drug solution filling step to a base solution drying step were performed in an environment with a temperature of 10°C and with a relative humidity of 35%RH.

### (Drug Solution Filling Step and Drug Solution Drying Step)

A drug solution filling step and a drug solution drying step were formed in the same way with Example 1.

### (Base Solution Filling Step and Base Solution Drying Step)

A base solution filling step and a base solution drying step were formed in the same way with Example 1.

### (Peeling-Off Step)

A peeling-off step was performed in the same way with Example 1.

### (Evaluation)

The amount of air bubbles generated in the needle-shaped projection in the produced transdermal absorption sheet was evaluated. As an evaluation method, the transdermal absorption sheet was observed using a microscope (VHX-600 manufactured by Keyence Corporation). The presence or absence of air bubble generation was evaluated in a plurality of transdermal absorption sheets produced, and there was no transdermal absorption sheet in which air bubble was present in the needle-shaped projections in the transdermal absorption sheets.

### <Example 3>

### (Production of Mold)

A mold was produced in the same way with Example 1.

### (Preparation of Solution containing Drug)

A solution was prepared in the same way with Example 1.

### (Preparation of Solution not containing Drug)

A solution was prepared in the same way with Example 1.

Hereinafter, a drug solution filling step to a base solution drying step were performed in an environment with a temperature of 1°C and with a relative humidity of 35%RH.

### (Drug Solution Filling Step and Drug Solution Drying Step)

A drug solution filling step and drug solution drying step were formed in the same way with Example 1.

### (Base Solution Filling Step and Base Solution Drying Step)

A base solution filling step and a base solution drying step were formed in the same way with Example 1.

### (Peeling-Off Step)

A peeling-off step was performed in the same way with Example 1.

### (Evaluation)

The amount of air bubbles generated in the needle-shaped projection in the produced transdermal absorption sheet was evaluated. As an evaluation method, the transdermal absorption sheet was observed using a microscope (VHX-600 manufactured by Keyence Corporation). The presence or absence of air bubble generation was evaluated in a plurality of transdermal absorption sheets produced, and there was no transdermal absorption sheet in which air bubble was present in the needle-shaped projections in the transdermal absorption sheets.

### <Example 4>

### (Production of Mold)

A mold was produced in the same way with Example 1.

### (Preparation of Solution containing Drug)

A solution was prepared in the same way with Example 1.

### (Preparation of Solution not containing Drug)

A solution was prepared in the same way with Example 1.

Hereinafter, a drug solution filling step to a base solution drying step were performed in an environment with a temperature of 5°C and with a relative humidity of 80%RH.

### (Drug Solution Filling Step and Drug Solution Drying Step)

A drug solution filling step and a drug solution drying step were formed in the same way with Example 1.

### (Base Solution Filling Step and Base Solution Drying Step)

A base solution filling step and a base solution drying step were formed in the same way with Example 1.

### (Peeling-Off Step)

A peeling-off step was performed in the same way with Example 1.

### (Evaluation)

The amount of air bubbles generated in the needle-shaped projection in the produced transdermal absorption sheet was evaluated. As an evaluation method, the transdermal absorption sheet was observed using a microscope (VHX-600 manufactured by Keyence Corporation). The presence or absence of air bubble generation was evaluated in a plurality of transdermal absorption sheets produced, and there was no transdermal absorption sheet in which air bubble was present in the needle-shaped projections in the transdermal absorption sheets.

### <Comparative Example>

### (Production of Mold)

A mold was produced in the same way with Example 1.

### (Preparation of Solution containing Drug)

A solution was prepared in the same way with Example 1.

### (Preparation of Solution not containing Drug)

A solution was prepared in the same way with Example 1.

Hereinafter, a drug solution filling step to a base solution drying step were performed in an environment with a temperature of 25°C and with a relative humidity of 35%RH.

### (Drug Solution Filling Step and Drug Solution Drying Step)

A drug solution filling step and a drug solution drying step were formed in the same way with Example 1.

### (Base Solution Filling Step and Base Solution Drying Step)

A base solution filling step and a base solution drying step were formed in the same way with Example 1.

### (Peeling-Off Step)

A peeling-off step was performed in the same way with Example 1.

### (Evaluation)

The amount of air bubbles generated in the needle-shaped projection in the produced transdermal absorption sheet was evaluated. As an evaluation method, the transdermal absorption sheet was observed using a microscope (VHX-600 manufactured by Keyence Corporation). The presence or absence of air bubble generation was evaluated in a plurality of transdermal absorption sheets produced, and there was present a slight number of transdermal absorption sheets in which air bubble was present in the needle-shaped projections in the transdermal absorption sheets.

### {Reference Signs List}

- 13:: MOLD
- 15:: NEEDLE-SHAPED RECESS
- 22:: DRUG SOLUTION
- 24:: BASE SOLUTION
- 34:: NOZZLE
- 34A:: LIP PORTION
- 34B:: OPENING PORTION
- 100:: TRANSDERMAL ABSORPTION SHEET
- 110:: NEEDLE-SHAPED PROJECTION
- 112:: NEEDLE PORTION
- 114:: FRUSTUM PORTION
- 116:: SHEET PORTION
- 120:: FIRST LAYER
- 122:: SECOND LAYER

## Claims

1. A method of manufacturing a transdermal absorption sheet (100) comprising, in this order:
a drug solution filling step of filling needle-shaped recesses (15) of a mold (13), the needle-shaped recesses (15) being arranged two-dimensionally, with a drug solution (22) that is a polymer solution containing a predetermined amount of a drug;
a drug solution drying step of drying the drug solution (22) filled in the needle-shaped recesses (15) to form a first layer (120) containing a predetermined amount of a drug;
a base solution filling step of filling the needle-shaped recesses (15) on the first layer (120) with a base solution (24) that is a polymer solution not containing a predetermined amount of a drug;
a base solution drying step of drying the base solution (24) to form a second layer (122) not containing a predetermined amount of a drug on the first layer (120); and
a peeling-off step of peeling off the first layer (120) and the second layer (122) from the mold (13), **characterized in that**
each step of at least from the drug solution filling step to the base solution drying step is performed in an environment with a temperature of 1°C to 10 °C.

2. The method of manufacturing a transdermal absorption sheet (100) according to claim 1,
wherein at least one step of the drug solution drying step and the base solution drying step is performed in an environment with a relative humidity of 1%RH to 59%RH.

3. The method of manufacturing a transdermal absorption sheet (100) according to claim 1 or 2, further comprising
at least one of a step of deaerating the drug solution (22) before the drug solution filling step and a step of deaerating the base solution (24) before the base solution filling step.

4. The method of manufacturing a transdermal absorption sheet (100) according to any one of claims 1 to 3,
wherein the needle-shaped recess (15) of the mold (13) has a through hole (15C) at a tip end thereof.

5. The method of manufacturing a transdermal absorption sheet (100) according to any one of claims 1 to 4,
wherein in the drug solution drying step, the drying is performed in a calm state.

6. The method of manufacturing a transdermal absorption sheet (100) according to any one of claims 1 to 5,
wherein in the drug solution filling step, a nozzle (34) that discharges the drug solution (22) is scanned on the mold (13) in a state where the nozzle (34) is pressed to the mold (13), and the needle-shaped recesses (15) are filled with the drug solution (22) from the nozzle (34) while a pressing force of the nozzle (34) to the mold (13) is controlled.

7. The method of manufacturing a transdermal absorption sheet (100) according to any one of claims 1 to 5,
wherein in the drug solution filling step, a nozzle (34) that discharges the drug solution (22) is scanned on the mold (13) in a state where the nozzle (34) is pressed to the mold (13), and the needle-shaped recesses (15) are filled with the drug solution (22) from the nozzle (34) while a push-in distance of the nozzle (34) to the mold (13) is controlled.

8. The method of manufacturing a transdermal absorption sheet (100) according to any one of claims 1 to 7,
wherein in the drug solution filling step, the amount of the drug solution (22) to be filled is equal to a total volume of the needle-shaped recesses (15).

9. The transdermal absorption sheet (100) according to any one of claims 1 to 8,
wherein the drug is a peptide, a protein, a nucleic acid, a polysaccharide, a vaccine, a medical compound belonging to a water-soluble low-molecular compound, or a cosmetic component.

## Patentansprüche

1. Verfahren zum Herstellen eines transdermalen Absorptionsflachstücks (100), umfassend in dieser Reihenfolge:
einen Arzneimittellösungs-Befüllungsschritt des Befüllens von nadelförmigen Vertiefungen (15) einer Form (13), wobei die nadelförmigen Vertiefungen (15) zweidimensional angeordnet sind, mit einer Arzneimittellösung (22), bei der es sich um eine eine vorbestimmte Arzneimittelmenge enthaltende Polymerlösung handelt;
einen Arzneimittellösungs-Trocknungsschritt des Trocknens der in die nadelförmigen Vertiefungen (15) gefüllten Arzneimittellösung (22), um eine erste Schicht (120) zu bilden, die eine vorbestimmte Menge eines Arzneimittels enthält;
einen Basislösungs-Befüllungsschritt des Befüllens der nadelförmigen Vertiefungen (15) der ersten Schicht (120) mit einer Basislösung (24), bei der es sich um Polymerlösung handelt, die nicht eine vorbestimmte Menge eines Arzneimittels enthält;
einen Basislösungs-Trocknungsschritt des Trocknens der Basislösung (24) zur Bildung einer zweiten Schicht (122), die nicht eine vorbestimmte Menge eines Arzneimittels enthält, auf der ersten Schicht (120); und
einen Abziehschritt des Abziehens der ersten Schicht (120) und der zweiten Schicht (122) von der Form (13),
**dadurch gekennzeichnet, dass**
jeder Schritt zumindest von dem Arzneimittellösungs-Befüllungsschritt bis zu dem Basislösungs-Trocknungsschritt in einer Umgebung mit einer Temperatur von 1 °C bis 10 °C ausgeführt wird.

2. Verfahren nach Anspruch 1, bei dem mindestens ein Schritt von dem Arzneimittellösungs-Trocknungsschritt und dem Basislösungs-Trocknungsschritt in einer Umgebung mit einer relativen Feuchtigkeit von 1 % RH bis 59 % RH ausgeführt wird.

3. Verfahren nach Anspruch 1 oder 2, weiterhin umfassend:
mindestens einen von einem Schritt des Entlüftens der Arzneimittellösung (22) vor dem Arzneimittellösungs-Befüllungsschritt und einem Schritt des Entlüftens der Basislösung (24) vor dem Basislösungs-Befüllungsschritt.

4. Verfahren nach einem der Ansprüche 1 bis 3,
bei dem die nadelförmige Vertiefung (15) der Form (13) ein Durchgangsloch (15C) an ihrem Ende aufweist.

5. Verfahren nach einem der Ansprüche 1 bis 4,
bei dem in dem Arzneimittellösungs-Trocknungsschritt das Trocknen in einem beruhigten Zustand erfolgt.

6. Verfahren nach einem der Ansprüche 1 bis 5,
bei dem in dem Arzneimittellösungs-Befüllungsschritt eine die Arzneimittellösung (22) austragende Düse (34) an der Form (13) in einem Zustand vorbeigeführt wird, in welchem die Düse (34) an die Form (13) gedrückt wird, und die nadelförmigen Vertiefungen (15) mit der Arzneimittellösung (22) aus der Düse (34) befüllt werden, während eine Andrückkraft der Düse (34) gegen die Form (13) gesteuert wird.

7. Verfahren nach einem der Ansprüche 1 bis 5,
bei dem in dem Arzneimittellösungs-Befüllungsschritt eine die Arzneimittellösung (22) austragende Düse (34) auf der Form (13) in einem Zustand geführt wird, in welchem die Düse (34) gegen die Form (13) gedrückt wird, und die nadelförmigen Vertiefungen (14) mit der Arzneimittellösung (22) aus der Düse (34) befüllt werden, während eine Eindrückdistanz der Düse (34) gegen die Form (13) gesteuert wird.

8. Verfahren nach einem der Ansprüche 1 bis 7,
bei dem in dem Arzneimittellösungs-Befüllungsschritt die Menge der einzufüllenden Arzneimittellösung (22) gleich einem Gesamtvolumen der nadelförmigen Vertiefungen (15) ist.

9. Transdermales Absorptionsflachstück (100) nach einem der Ansprüche 1 bis 8,
bei dem das Arzneimittel ein Peptid, ein Protein, eine Nukleinsäure, ein Polysaccharid, ein Impfstoff, eine medizinische Verbindung zu einer wasserlöslichen, niedermolekularen Verbindung oder eine kosmetische Komponente ist.

## Revendications

1. Procédé de fabrication d'une feuille d'absorption transdermique (100), comprenant, dans cet ordre :
une étape de remplissage de solution médicamenteuse, pour remplir des retraits en forme d'aiguille (15) d'un moule (13), les retraits en forme d'aiguille (15) étant agencés de manière bidimensionnelle, avec une solution médicamenteuse (22) qui est une solution polymère contenant une quantité prédéterminée d'un médicament ;
une étape de séchage de solution médicamenteuse, pour sécher la solution médicamenteuse (22) remplie dans les retraits en forme d'aiguille (15) pour former une première couche (120) contenant une quantité prédéterminée d'un médicament ;
une étape de remplissage de solution de base, pour remplir les retraits en forme d'aiguille (15) sur la première couche (120) avec une solution de base (24) qui est une solution polymère ne contenant pas une quantité prédéterminée d'un médicament ;
une étape de séchage de solution de base, pour sécher la solution de base (24) pour former une seconde couche (122) ne contenant pas une quantité prédéterminée d'un médicament sur la première couche (120), et
une étape de pelage, pour peler la première couche (120) et la seconde couche (122) hors du moule (13),
**caractérisé en ce que**
chaque étape d'au moins les étapes allant de l'étape de remplissage de solution médicamenteuse à l'étape de séchage de solution de base est réalisée dans un environnement à une température allant de 1 °C à 10 °C.

2. Procédé de fabrication d'une feuille d'absorption transdermique (100) selon la revendication 1,
dans lequel au moins une étape parmi l'étape de séchage de solution médicamenteuse et l'étape de séchage de solution de base est réalisée dans un environnement présentant une humidité relative de 1% RH à 59 % RH.

3. Procédé de fabrication d'une feuille d'absorption transdermique (100) selon la revendication 1 ou 2, comprenant en outre :
au moins une étape parmi une étape de désaération de la solution médicamenteuse (22) avant l'étape de remplissage de solution médicamenteuse et une étape de désaération de la solution de base (24) avant l'étape de remplissage de solution de base.

4. Procédé de fabrication d'une feuille d'absorption transdermique (100) selon l'une quelconque des revendications 1 à 3,
dans lequel le retrait en forme d'aiguille (15) du moule (13) présente un trou débouchant (15C) sur une extrémité de pointe de celui-ci.

5. Procédé de fabrication d'une feuille d'absorption transdermique (100) selon l'une quelconque des revendications 1 à 4,
dans lequel lors de l'étape de séchage de solution médicamenteuse, le séchage est réalisé dans un état calme.

6. Procédé de fabrication d'une feuille d'absorption transdermique (100) selon l'une quelconque des revendications 1 à 5,
dans lequel lors de l'étape de remplissage de solution médicamenteuse, une buse (34) qui libère la solution médicamenteuse (22) est balayée sur le moule (13) dans un état où la buse (34) est pressée vers le moule (13), et les retraits en forme d'aiguille (15) sont remplis de solution médicamenteuse (22) à partir de la buse (34) tandis qu'une force de pression de la buse (34) vers le moule (13) est contrôlée.

7. Procédé de fabrication d'une feuille d'absorption transdermique (100) selon l'une quelconque des revendications 1 à 5,
dans lequel lors de l'étape de remplissage de solution médicamenteuse, une buse (34) qui libère la solution médicamenteuse (22) est balayée sur le moule (13) dans un état où la buse (34) est pressée vers le moule (13), et les retraits en forme d'aiguille (15) sont remplis de solution médicamenteuse (22) à partir de la buse (34) tandis qu'une distance de poussée de la buse (34) vers le moule (13) est contrôlée.

8. Procédé de fabrication d'une feuille d'absorption transdermique (100) selon l'une quelconque des revendications 1 à 7,
dans lequel lors de l'étape de remplissage de solution médicamenteuse, la quantité de la solution médicamenteuse (22) à remplir est égale à un volume total des retraits en forme d'aiguille (15).

9. Feuille d'absorption transdermique (100) selon l'une quelconque des revendications 1 à 8,
dans laquelle le médicament est un peptide, une protéine, un acide nucléique, un polysaccharide, un vaccin, un composé médical appartenant à un composé moléculaire à faible poids moléculaire et soluble dans l'eau, ou un composant cosmétique.
